# EUROPEAN PATENT SPECIFICATION

(11) **EP 1 824 520 B1**
(45) Date of publication and mention of the grant of the patent: **27.04.2016**
(21) Application number: 05803689.8
(22) Date of filing: 16.11.2005
(51) Int. Cl.: G01N 33/60, G01N 33/574, A61K 51/04

(54) **METHODS OF DETECTING PROSTATE CANCER**
VERFAHREN ZUM NACHWEIS VON PROSTATAKARZINOM
METHODES POUR DETECTER LE CANCER DE LA PROSTATE

(30) Priority: 17.11.2004 US 628105 P; 29.04.2005 US 675892 P
(43) Date of publication of application: 29.08.2007
(73) Proprietor: Biosensors International Group, Ltd., Hamilton HM 11 (BM)
(72) Inventor: DICKMAN, Dalia, 20184 Doar-Na Misgav (IL)
(74) Representative: Dennemeyer & Associates S.A.
(86) International application number: PCT/IL2005/001215
(87) International publication number: WO 2006/054296

(56) References cited:
- US-B1- 6 261 562
- MAO P ET AL: "Human prostatic carcinoma: an electron microscope study.", CANCER RESEARCH MAY 1966 LNKD- PUBMED:5934805, vol. 26, no. 5, May 1966 (1966-05), pages 955-973, XP002625777, ISSN: 0008-5472
- HERRMANN PAUL C ET AL: "Mitochondrial proteome: Altered cytochrome c oxidase subunit levels in prostate cancer.", PROTEOMICS, vol. 3, no. 9, September 2003 (2003-09), pages 1801-1810, XP002625778, ISSN: 1615-9853
- KRIEG RENE C ET AL: "Mitochondrial proteome: Cancer-altered metabolism associated with cytochrome c oxidase subunit level variation", PROTEOMICS, vol. 4, no. 9, September 2004 (2004-09), pages 2789-2795, XP002625779, ISSN: 1615-9853
- STOREY G R ET AL: "Tc-99m sestamibi uptake in metastatic prostate carcinoma.", CLINICAL NUCLEAR MEDICINE FEB 2000 LNKD- PUBMED:10656651, vol. 25, no. 2, February 2000 (2000-02), pages 133-134, XP009145398, ISSN: 0363-9762

## Description

The present invention relates to methods of detecting prostate cancer. More particularly, the present invention relates to a method of correlating the quantity and/or characteristic of prostate cell-mitochondria or a prostate cell mitochondrial component to the presence/absence or state of prostate cancer in a subject.

Prostate cancer is the most common solid tumor and the second leading cause of cancer deaths among men in the United States [Landis et al., CA Cancer J. Clin. 49:8-31 (1999)]. The prevalence of prostate cancer varies worldwide with the highest frequency found in African Americans and the lowest frequency found in Asian populations [Parkin et al., Int. J. Cancer 54:594-606 (1993)]. According to the American Cancer Society, there were an estimated 230,110 new cases for prostate cancer in the United States in 2004 and 29,900 estimated deaths from prostate cancer in the United States in 2004.

The presently known methods of treating prostate cancer mainly involve radiotherapy, surgery or hormone therapy. The treatment options for prostate cancer depend in part on whether the tumor has spread and the rate at which it is growing. For in-situ tumors which have not yet metastasized, radiotherapy and radical prostatectomy, involving the surgical removal of the whole prostate and the nearby lymph nodes, are the presently most common treatment options. Although surgery offers the most certain treatment, it is accompanied by adverse side effects. Apart from the obvious psychological side effects, the main risks of prostatectomy include incontinence and impotence.

Currently practiced radiotherapy protocols for treating prostate cancer are accompanied by adverse side effects such as impotence and frequently do not lead to complete abolishment of the tumor. At 10 years post-treatment, cure rates are about 79 % for both radiotherapy and for radical prostatectomy individually.

Generally, tumors that have grown beyond the edge of the prostate cannot be cured with either radiation or surgery and must be treated with hormones to slow the cancer's growth. While prostate cancer usually responds to one or two years of hormone therapy, after some time most tumors will become resistant to therapy and re-grow. The only treatment remaining is symptom control.

Since the above treatment therapies cannot cure prostate cancer once it has spread beyond the gland, treatment of localized tumors is the best hope for lowering the mortality rate for prostate cancer. Thus, early detection and diagnosis of prostate cancer is critical for disease management.

At present, the first steps in diagnosing symptomatic or non-symptomatic prostate cancer utilize two standard screening examinations. The first is a digital rectal examination (DRE), in which a doctor inserts a gloved finger into the rectum to feel the prostate gland through the rectal wall to check for lumps or abnormal areas. Although this test has been used for many years, its effectiveness in decreasing the number of deaths from prostate cancer is questionable.

The second standard screening examination is a routine blood test to detect the amount of prostate-specific antigen (PSA) circulating in the blood. PSA is a marker that, if present in higher than average amounts, typically above 4ng/ml, may indicate the presence of prostate cancer cells. However, since prostate cancer has been detected also with PSA levels lower than 4.0 ng/ml and further since it was found that PSA levels may be higher in men who have non-cancerous prostate conditions, false positives as well as false negatives have been associated with this screening regime, reducing its credibility.

Hence, since diagnosing prostate cancer by an abnormal-feeling prostate and/or an elevated PSA level do not provide a definitive diagnosis, biopsies of cancerous tissue samples taken from the prostate gland are always required for definitive diagnosis. To that end, a prostate biopsy method known as the sextant biopsy is the presently preferred diagnosis method [Hodge et al J Urol, 142: 71, 1989]. In this prostate biopsy method, an average of six cores are taken from the prostate (top, middle and bottom; right and left sides), so as to obtain a representative sample of the prostate gland and to determine the presence of malignant cells. The biopsies are examined and if prostate cancer is diagnosed its aggressiveness is determined using the Gleason grading system. This system provides an estimate of the cancer's potential to grow and spread to other parts of the body. In general, a high Gleason grade (greater than or equal to 7.0) indicates an aggressive prostate tumor that is likely to spread to other organs.

A prostate biopsy is typically performed in conjunction with a trans-rectal ultrasound (TRUS) probe in order to provide pictures of the prostate during the biopsy procedure and to guide the precise placement of the biopsy needle. In this procedure, high-frequency sound waves are sent out by a probe, which is inserted into the rectum. The waves bounce off the prostate gland and produce echoes from which a computer-generated sonogram is obtained. The sonogram is examined for echoes that might represent abnormal areas such as prostate cancer foci, based on contrast between the cancerous mass and normal prostate tissue. This contrast, however, can be visualized only when a relatively large cancerous mass is present and even then it is very subtle and can be easily missed by the practitioner. While transrectal ultrasound imaging of the prostate is the golden standard, it only provides anatomical imaging of the prostate boundaries during the biopsy procedure.

However, studies have demonstrated that the sextant technique for obtaining prostate biopsy underestimates the presence of prostate cancer. Repeated transrectal ultrasound guided sextant prostate biopsies may detect prostate cancer in 19-28 % of patients with an initially negative biopsy [Roehrborn, C. G. et al., Urology, 47: 347, 1996; Ellis, W.J. et al., J. Urol., 153: 1496, 1995; Keetch, D. W. et al., J. Urol., 151: 1571, 1994; Fleshner, N.E. et al., J. Urol., 157: 556, 1997].

Furthermore, in a study carried out by Rabbani *et al,* which was aimed at assessing the incidence and clinical significance of false negative sextant prostate biopsies in patients undergoing radical prostatectomy, it was noted that of the 118 patients, 27 (23 %) had a false negative repeat transrectal ultrasound guided sextant prostate biopsy [Rabbani F. et al., J Urol. 1998 Apr;159(4):1247-50].

Although multiple in vivo studies have revealed that increasing the number of prostate biopsies enhances prostate cancer detection, this is associated with increased cost, and potential morbidity with diminishing benefit.

In view of the above, there is a widely recognized need for and it would be highly advantageous to have an improved method of diagnosing prostate cancer, which would aid in accurate staging of the tumor and as a result give more confidence for choosing the best treatment regime for each patient as well as provide a tool for differential diagnosis of recurrent cancer.

Cancer cells, in general, have an altered metabolism including a higher rate of glycolysis, an increased rate of glucose transport, increased gluconeogenesis, reduced pyruvate oxidation and increased lactic acid production, increased glutaminolytic activity, reduced fatty acid oxidation, increased glycerol and fatty acid turnover, modified amino acid metabolism, and increased pentose phosphate pathway activity.

Mitochondria are involved either directly or indirectly in many aspects of altered metabolism in cancer cells. Mitochondria are found in eukaryotic cells, constituting approximately 10 % of the cell volume. They are pleomorphic organelles with structural and numerical variations depending on cell type, cell-cycle stage and intracellular metabolic state. The key function of mitochondria is energy production through oxidative phosphorylation (OxPhos) and lipid oxidation and notable differences between the mitochondria of normal versus transformed cells have been discovered [Carafoli, E. (1980) Mol. Aspects Med. 3, 295-429]. For example, various tumor cell lines exhibit differences in the number, size and shape of their mitochondria relative to normal controls. The mitochondria of rapidly growing tumors tend to be fewer in number, smaller and have fewer cristae than mitochondria from slowly growing tumors; the latter are larger and have characteristics more closely resembling those of normal cells. On the other hand, oncocytoma of thyroid, salivary gland, kidney, parathyroid and breast are characterized by the presence of cells containing abnormally large numbers of mitochondria, and high levels of oxidative enzymes [Maximo, V. et al., (2000) Virchows Arch 437, 107-115]. The ultrastructural features of mitochondria in these cells show similarities with mitochondrial encephalomyopathies, where mitochondria are found as large aggregates and display a variety of morphological alterations [Maximo, V. et al., (2000) Virchows Arch 437, 107-115].

Alterations in the molecular composition of the inner membranes of tumor mitochondria have also been noted [Chang, et al., (1971) Cancer Res. 31, 108-113]. Polypeptide profiles of normal liver versus hepatoma mitochondria demonstrate differences in the appearance and/or relative abundance of several protein subunits. One major band that is deficient or absent in several tumors studied has a mobility near or equal to the B subunit of the F₁-ATPase (approximately 57 kDa). Other bands that are present in tumor mitochondria appear to be deficient or absent in control mitochondria. In addition, analysis of the inner membrane lipid composition of various tumor mitochondria has indicated elevated levels of cholesterol, varying total phospholipid content, and/or changes in the amount of individual phospholipids relative to normal controls.

Differences in the mitochondria of normal versus transformed cells have also been noted with regard to: (1) the preference for substrates oxidised; (2) the magnitude of the acceptor control ratio; (3) the rates of electron and anion transport; (4) the capacity to accumulate and retain calcium; (5) the amounts and forms of DNA; (6) the rates of protein synthesis and organelle turnover and (7) mitochondrial surface potential (DELTA..PSI.m). However, there is apparently no universal mitochondrial metabolic alteration that is common to all tumors. For example, although the pathogenesis of prostate cancer involves the mitochondrial metabolic transformation of citrate-producing cells to citrate-oxidising cells, this metabolic abnormality is not reported in other cancers [Costello, L.C. and Franklin, R.B. (2000) Oncology 59, 269-282].

Several other distinct differences between the mitochondria of normal cells and cancer cells have been observed at the microscopic, molecular, biochemical, metabolic and genetic levels. Differential expression of mitochondrial cytochrome oxidase II in benign and malignant breast tissues has been reported [Sharp et al., J Pathol 1992, 168:163-168]. Furthermore, mutations in mitochondrial DNA (mtDNA) are commonly found in a variety of cancers including the ovarian, thyroid, salivary, kidney, liver, lung, colon, gastric, brain bladder, head and neck, leukemia and breast cancers [Penta et al., Mutat Res 2001,488:119-133].

"Human Prostatic Carcinoma: An Electron Microscope Study", by Mao, P., et al., in Cancer Research, vol. 26, no. 9, May 1966, pages 955 - 973, discloses that "Electron microscopic studies of human prostatic cancers revealed that cytologically well-differentiated and poorly differentiated tumor cells are present in both histologically well-differentiated and anaplastic prostatic cancers.". Also discloses that "Outstanding features of prostatic cancer cells, when compared to normal and hyperplastic prostatic epithelial cells, are the nuclear hypertrophy, hyperchromatinism, nucleolar hypertrophy, increase in number and pleomorphism of mitochondria,", among other features.

"Mitochondrial proteome: Altered cytochrome c oxidase subunit levels in prostate cancer", by Herrmann, P.C., et al., in Proteomics 2003, vol. 3, no. 9, September 2003, pages 1801 - 1810, discloses that "Laser capture microdissection was combined with reverse phase protein lysate arrays to quantitatively analyze the ratios of mitochondrial encoded cytochrome c oxidase subunits to nuclear encoded cytochrome c oxidase subunits, and correlating the ratios with malignant progression in human prostate tissue specimens.".

"Mitochondrial proteome: Cancer-altered metabolism associated with cytochrome c oxidase subunit level variation", by Krieg, R.C., et al., in Proteomics 2004, vol. 4, no. 9, September 2004, pages 2789 - 2795, discloses about "testing the hypothesis that there is a specific association between altered cytochrome c oxidase subunit levels and altered metabolism by combining the technique of reverse-phase protein microarray with radiolabeled glucose metabolic studies.". Also discloses that "there is an associated increase in the ratio of nuclear encoded cytochrome c oxidase subunits to mitochondrially encoded cytochrome c oxidase subunits in the tumor-derived cell lines.".

"Tc-99m Sestamibi Uptake in Metastatic Prostate Carcinoma", by Storey, G.R., et al., in Clinical Nuclear Medicine, vol. 25, no. 2, February 2000, pages 133 - 134, discloses that "Increased sestamibi uptake in an irregular pattern in multiple ribs was noted incidentally". Also discloses that "A whole-body bone scan showed corresponding multiple foci of increased tracer accumulation in the ribs and elsewhere in the axial and appendicular skeleton, consistent with widespread prostatic carcinoma metastases in bone."

In summary, it is recognized that genetic and/or metabolic alterations in mitochondria are associated with cancer, either as contributory or resulting factors. Several distinct differences between the mitochondria of normal cells and cancer cells have been observed at the genetic, structural, numerical, molecular and biochemical levels. However, not one single mitochondrial alteration is predictive of all kinds of cancer. Although established for several other cancers, as yet mitochondrial changes have never been established as a diagnostic marker for prostate cancer.

### SUMMARY OF THE INVENTION

According to one aspect of the present invention there is provided a method of obtaining information suitable for detecting presence or absence of early stage prostate cancer, being stage I or II prostate cancer, in the prostate of a subject after in vivo administration of a Tc^{99m}Sestamibi radioimaging agent to the subject, the method comprising reviewing and processing previously obtained results of radioimaging at least a portion of the prostate of the subject; ex vivo histochemically staining at least one prostate cell of the prostate with a reagent capable of binding to, or accumulating in, mitochondria; analyzing mitochondria or a mitochondrial component of the at least one prostate cell; corroborating the results of radioimaging with the ex vivo histochemical staining; and measuring an alteration in quantity and/or characteristic of the mitochondria or mitochondrial component with respect to a normal prostate cell of the prostate.

According to further features in preferred embodiments of the invention described below, the mitochondrial component is selected from the group consisting of mitochondrial protein, mitochondrial glycoprotein, mitochondrial lipid, mitochondrial peptide and mitochondrial nucleic acid.

According to still further features in the described preferred embodiments the mitochondrial protein is an enzyme or a structural protein.

According to still further features in the described preferred embodiments the characteristic of the mitochondria is selected from the group consisting of mitochondrial size, mitochondrial mass, mitochondrial potential and mitochondrial volume.

According to still further features in the described preferred embodiments the reagent is labeled with a detectable moiety.

According to still further features in the described preferred embodiments the detectable moiety is selected from the group consisting of a polypeptide and a chemical.

According to still further features in the described preferred embodiments the polypeptide is selected from the group consisting of an enzyme, a fluorescent polypeptide and an epitope.

According to still further features in the described preferred embodiments the chemical is a radioactive isotope, a phosphorescent chemical, a chemiluminescent chemical and a fluorescent chemical.

According to still further features in the described preferred embodiments the radioactive isotope can be detected by radioimaging.

According to still further features in the described preferred embodiments the radioactive isotope is selected from the group consisting of Technetium^{99m}, Carbon¹¹, Oxygen¹⁵, Nitrogen¹³, Rubidium⁸², Gallium⁶⁷, Gallium⁶⁸, Yttrium⁹⁰, Molybdenum⁹⁹, Iodine^{123,124,131} Fluorine¹⁸,, Phosphorus³², Copper⁶², Thallium²⁰¹, Copper⁶⁴, Copper⁶², Indium¹¹¹, Xenon¹³³.

According to still further features in the described preferred embodiments the reagent is selected from the group consisting of Tc⁹⁹Sestamibi, Tc⁹⁹-tetrofosmin, Tc⁹⁹-triphenylalkylphosphonium, ¹⁸fluorine-labeled-2-fluoro-2-deoxyglucose, ⁶²Cu-diacetyl-bis(N4-methylthiosemicarbazone) and ⁶⁴Cu-1,4,8,11-tetraazacyclotetradecane-N,N',N",N"'-tetraacetic acid - octreotide.

According to still further features in the described preferred embodiments the method further comprises imaging the at least one ex vivo histochemically stained prostate cell.

According to still further features in the described preferred embodiments the imaging is selected from the group consisting of radioimaging, fluorescence imaging, color imaging, biophotonic imaging and magnetic resonance imaging.

According to still further features in the described preferred embodiments the radioimaging is selected from the group consisting of single photon emission computed tomography (SPECT), positron emission tomography (PET) and gamma cameras.

According to still further features in the described embodiments, the radioimaging provides one or more images of one or more sections or slices of the prostate.

According to still further features in the described preferred embodiments the at least one prostate cell is intact.

According to still further features in the described preferred embodiments the at least one prostate cell is disintegrated.

According to still further features in the described preferred embodiments the reagent is selected from the group consisting of a chemical, a dye, a peptide, a polypeptide and a polynucleotide.

According to still further features in the described preferred embodiments the dye is administered directly into the at least one prostate cell.

According to still further features in the described preferred embodiments the dye is membrane potential-independent.

According to still further features in the described preferred embodiments the membrane potential-independent dye is selected from the group consisting of nonyl acridine orange, MitoTracker Green FM, MitoFluor Green and MitoFluor Red 589.

According to still further features in the described preferred embodiments the dye is membrane potential-dependent.

According to still further features in the described preferred embodiments the membrane potential-dependent dye is selected from the group consisting of MitoTracker Orange CMTMRos, MitoTracker Orange CM-H₂TMRos, MitoTracker Red CMXRos, MitoTracker Red CM-H₂XRos, MitoTracker Red 580, MitoTracker Deep Red 633, MitoFluor Red 594, RedoxSensor Red CC-1 (2,3,4,5,6-pentafluorotetramethyldihydrorosamine, JC-1 probe (5,5',6,6'-tetrachloro-1,1',3,3'-tetraethylbenzimidazolylcarbocyanine iodide, Rhodamine 123, tetramethylrosamine, rhodamine 6G, tetramethylrhodamine methyl ester, tetramethylrhodamine ethyl ester, dihydrorhodamine, dihydrotetramethylrosamine, DiOC₂(3), DiOC₅(3), DiOC₆(3), DiSC₃(5), DiIC₁(5), DASPMI (4-Di-1-ASP), DASPEI and CoroNa Red Na⁺.

According to still further features in the described preferred embodiments the polypeptide is selected from the group consisting of an antibody an avidin and a derivative thereof.

According to still further features in the described preferred embodiments the avidin derivative is selected from the group consisting of avidin, strepavidin and nutravidin.

According to still further features in the described embodiments, the corroborating comprises analyzing photographs of the radioimaging and the ex vivo histochemical staining.

According to still further features in the described embodiments, the corroborating comprises correlating areas of the radioimaged prostate portion and the at least one ex vivo histochemically stained prostate cell.

The present invention successfully addresses the shortcomings of the presently known configurations by providing novel methods of identifying prostate cancer.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

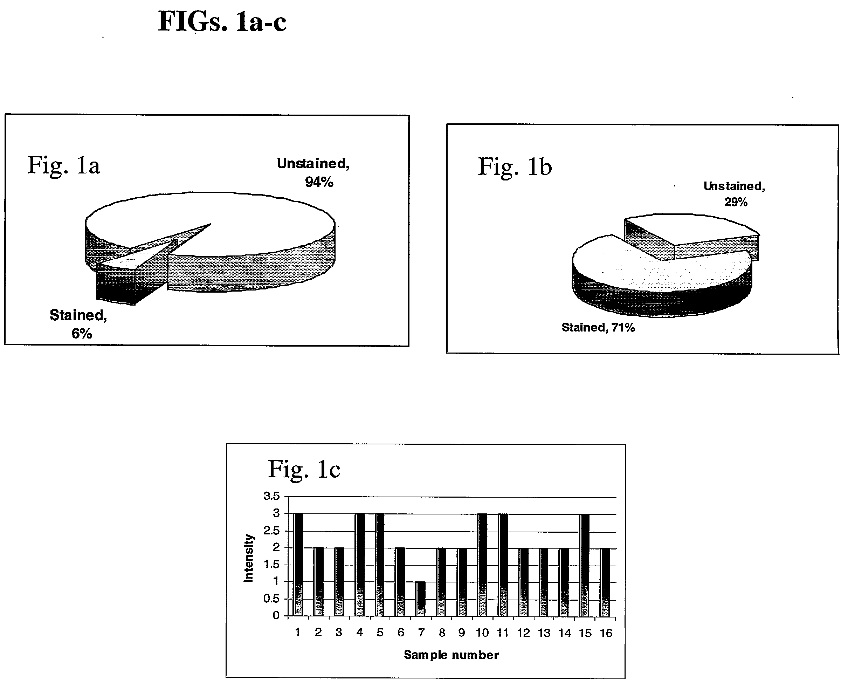

FIGs.1a-c are graphical representations of the statistical data relating to the immunohistochemistry of prostate cancer tissue: Figure 1a is a pie chart depicting the ratio of stained normal cells to unstained normal cells; Figure 1b is a pie chart depicting the ratio of stained tumor cells to unstained tumor cells; Figure 1c is a bar graph depicting the staining intensity of tumor cells.

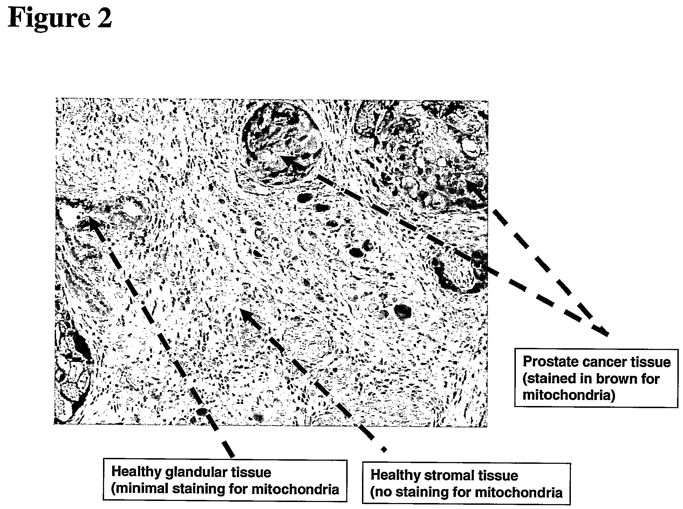

FIG. 2 is a photograph of prostate cancer cells immunohistochemically stained with a mitochondrial specific antibody.

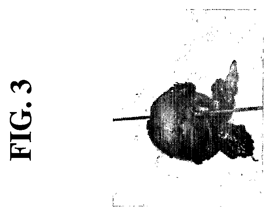

FIG. 3 is a photograph of an excised intact prostate by radical retropubic prostatectomy following in vivo Tc⁹⁹Sestamibi administration.

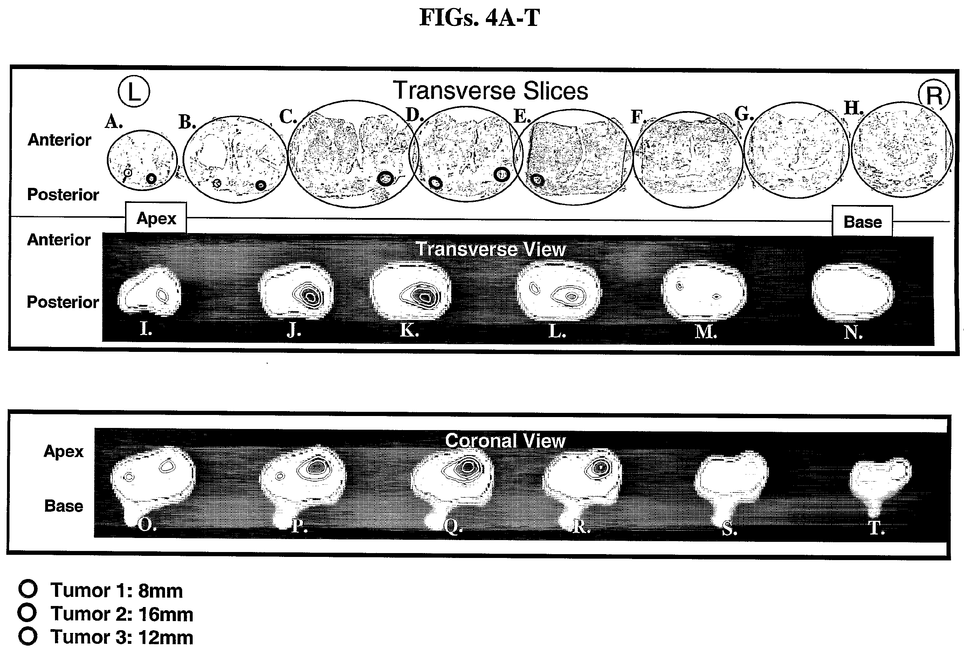

FIGs. 4A-T are photographs of whole mount histopathology analysis of prostate slices and SPECT scanning results in transaxial & coronal slices of an excised intact prostate following in vivo Tc⁹⁹Sestamibi administration. Figures 4A-H are a sequential series of whole mount histopathology analysis using a mitochondrial specific antibody on traversely sliced prostate tissue from a first patient. Figures 4I-N depict SPECT scanning results of prostate transaxial slices. Figures 4O-T depict SPECT scanning results of prostate coronal slices. The most intense red light from the SPECT scanning images shows the site of the tumor.

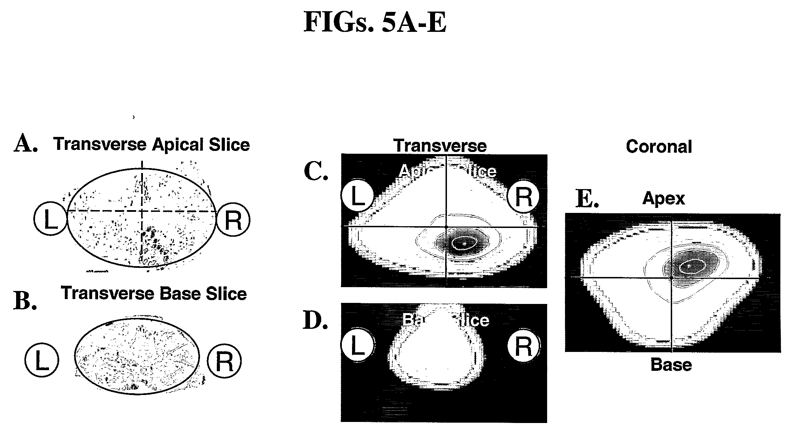

FIGs. 5A-E are photographs of whole mount histopathology analysis of prostate slices and SPECT scanning results in transaxial & coronal slices of an excised intact prostate from a second patient following in vivo Tc⁹⁹Sestamibi administration. Figures 5A-B are whole mount histopathology analyses using a mitochondrial specific antibody on traversely sliced prostate tissue from a first patient. Figures 5C-D depict SPECT scanning results of prostate transaxial slices. Figures 5E depicts SPECT a scanning result of a prostate coronal slice. The most intense red light from the SPECT scanning images shows the site of the tumor.

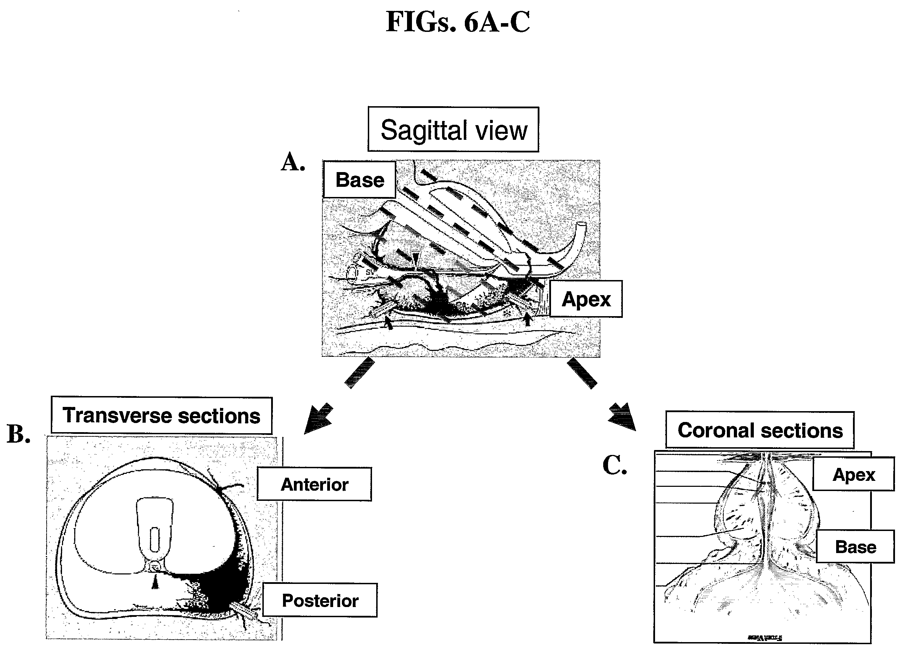

FIGs. 6A-C are anatomical drawings illustrating the prostate orientation of slices. Figure 6A is a sagittal view of the prostate. Figure 6B illustrates the orientation of transverse sections. Figure 6C illustrates the orientation of coronal sections.

### DESCRIPTION OF THE PREFERRED EMBODIMENTS

The present disclosure is of methods of detecting prostate cancer cells, which can be used for prostate cancer diagnosis both in vivo and ex vivo.

The principles of the indicative markers for prostate cancer according to the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Prostate cancer has evolved as a major health problem in the male population of the Western world. It is the most commonly diagnosed malignancy and the second leading cause of cancer death, representing nearly 29 % of all male cancer deaths. In the year 2001, about 200,000 new cases of prostatic adenocarcinomas were projected in the United States. Early diagnosis of the disease is crucial for disease management and improving prognosis.

Current methods of diagnosing prostate cancer often do not provide a definitive diagnosis at early stages of the disease.

Mitochondrial dysfunction is one of the most profound features of cancer cells. For example, the mitochondria of rapidly growing tumors tend to be fewer in number, smaller and have fewer cristae than mitochondria from slowly growing tumors; the latter are larger and have characteristics more closely resembling those of normal cells. On the other hand, other types of cancer such as breast cancer are characterized by the presence of cells containing abnormally large numbers of mitochondria, and high levels of oxidative enzymes. Other examples of mitochondrial changes specific to a particular cancer include the differential expression of mitochondrial cytochrome oxidase II in benign and malignant breast tissues [Sharp et al., J Pathol 1992, 168:163-168]. Mitochondrial DNA mutations, specific to a particular cancer are also commonly found in a range of cancers [Penta et al., Mutat Res 2001,488:119-133].

However, there is no universal mitochondrial alteration and thus no mitochondrial marker, which is common to all tumors. Alterations in mitochondria or mitochondrial components have never been suggested as a diagnostic marker for prostate cancer.

While reducing the present invention to practice, the present inventors have shown, for the first time, that analyzing mitochondria or mitochondrial components of prostate cells can lead to accurate diagnosis of cancerous prostate cells.

As is illustrated in the Examples section which follows, the present inventors have shown that cancer cells can be detected by immunohistochemical staining using a mitochondrial specific antibody.

Altogether these findings show that, prostate cancer cells can be differentiated from normal cells by mitochondrial analysis suggesting that mitochondrial analysis can be applied towards accurate diagnosis and staging of prostate cancer both at early and later stages. Thus, the present invention can serve as a valuable diagnostic tool for prostate cancer, which can be critical in determining the appropriate treatment course.

Thus, according to one aspect of the present disclosure, there is provided a method of detecting the presence or absence of prostate cancer in a subject. The method comprises analyzing mitochondria or a mitochondrial component of at least one prostate cell such that an alteration in quantity of a mitochondria or a mitochondrial component and/or a characteristic of a mitochondria with respect to a normal prostate cell is indicative of the presence or absence of the prostate cancer in the subject.

As used herein, "prostate cancer" is defined as cancer of the prostate gland, typically adenocarcinoma of the prostate gland.

As used herein a "subject" refers to a mammal, preferably a human subject. Examples of non-human mammals include, but are not limited to, mouse, rat, rabbit, bovine, porcine, ovine, canine and feline. The subject may show symptoms of prostate cancer or may be asymptomatic. Since the known treatment therapies cannot cure prostate cancer once it has spread beyond the gland, treatment of localized tumors is the best hope for lowering the mortality rate for prostate cancer. Accordingly, one of the primary foci of prostate cancer management should be early detection and diagnosis, so as to avoid a non-localized stage of prostate cancer. Therefore, the diagnosed subject preferably has an early stage of prostate cancer such as stage I or II prostate cancer. As used herein "stage I prostate cancer" is when the tumor is microscopic and confined to the prostate. It is typically undetectable by a digital rectal exam (DRE) or by ultrasound and usually discovered by PSA tests and subsequent biopsies. Stage II prostate cancer is when the tumor is confined to the prostate and typically can be detected by DRE or ultrasound. In stage III prostate cancer, the cancer has broken through the covering of the prostate and may have grown into the neck of the bladder or the seminal vesicle. Stage IV cancer is typically characterized by metastasis to another part of the body.

Prostate cancer is considered "present" if the quantity of a mitochondria or a mitochondrial component is altered and/or a mitochondrial characteristic is altered [i.e., statistically significant, as can be determined by analysis of variance (e.g., t-Test)] per cell compared with non-cancerous control prostate cells ("normal cell", further described herein below).

As used herein, the phrase "mitochondrial component" refers to a moiety which is present (e.g., expressed) only in mitochondria. Examples of mitochondrial components include but are not limited to mitochondrial proteins, mitochondrial glycoproteins, mitochondrial lipids, mitochondrial peptides and mitochondrial nucleic acids.

Examples of mitochondrial proteins include, but are not limited to proteins in the oxidative phosphorylation system, proteins in the oxidative phosphorylation complex I, II, III and IV, mitochondrial porin (VDAC1) GeneID 7416 and pyruvate dehydrogenase (ACAT1) GeneID 38. Endogenously biotinylated proteins in mammalian cells are present almost exclusively in mitochondria, where biotin synthesis occurs and thus serve as a further example of a protein mitochondrial component.

An example of a specific mitochondrial glycoprotein is C1QBP complement component 1, q subcomponent binding protein (GneID 708) [Tye et al., J. Biol. Chem., Vol. 276, Issue 20, 17069-17075, May 18, 2001].

As mentioned above, mitochondrial DNA is an example of a mitochondrial component. The human mitochondrion contains 5-10 identical, circular molecules of DNA. Each consists of 16,569 base pairs carrying the information for 37 genes which encode two different molecules of ribosomal RNA (rRNA) 22 different molecules of transfer RNA (tRNA) and 13 polypeptides.

Cardiolipin (1,3-diphosphatidylglycerol) is an example of a lipid mitochondrial component [Kirkland et al.,Neuroscience. 2002;115(2):587-602].

As used herein, the phrase "characteristic of a mitochondria" comprises the size of a mitochondria, the mass of a mitochondria, the volume of a mitochondria or the potential across the inner and outer membrane of a mitochondria. In the mitochondrial respiratory pathway, an electrochemical gradient is generated by the active extrusion of protons from the mitochondrial matrix to the intermembrane space via complexes of the electron transport chain. This inner membrane potential is a specific mitochondrial property of actively respiring mitochondria which attracts some agents of the present invention as detailed herein below. The characteristic of a mitochondria may reflect a change in quantity of a mitochondrial component or a change in activity of a mitochondrial component (e.g. a mitochondrial enzyme).

Methods of analyzing alterations in these characteristics are further detailed herein below.

As used herein, the phrase "a normal prostate cell" refers to a prostate cell taken from a healthy subject or from unaffected prostate tissue of the same subject. Since the above-mentioned mitochondrial characteristics and quantity depend on, amongst other things, species, age and cell type, it is preferable that the non-cancerous control cells come from a subject of the same species, age and from the same sub-prostate tissue. Alternatively, control data may be taken from databases and literature. It is preferable that at least one cell is analyzed in accordance with the present invention. However the present invention certainly envisages the analysis of few cells to millions of cells.

As used herein, the phrase "analyzing mitochondria or a mitochondrial component" refers to measuring a quantitative or qualitative difference between the mitochondria or mitochondrial component of a prostate cell of the test subject and a normal prostate cell.

Analyzing mitochondria according to this aspect of the present invention can be effected by exposing the cell to an agent capable of binding to, or accumulating in mitochondria. As mentioned, the agent may bind to, accumulate in, or be attracted by any mitochondrial specific component or characteristic of the mitochondria. Such an agent can be a chemical, a dye, a peptide, polypeptide (such as a mitochondria specific antibody) and a polynucleotide which may bind to mitochondria specific DNA as further described hereinbelow.

Detailed examples of agents which can be used in iv-vivo and ex-vivo applications are further described hereinbelow.

The mitochondrial agents may be labeled with a detectable moiety which can be quantified either directly or indirectly. Examples of detectable moieties that can be used in the present invention include but are not limited to radioactive isotopes, phosphorescent chemicals, chemiluminescent chemicals, fluorescent chemicals, enzymes, fluorescent polypeptides, and epitope tags.

As used herein in the specification and claims section that follows, the phrase "radioactive isotope" is an isotopic form of an element with an unstable nucleus that stabilizes itself by emitting ionizing radiation. Examples of radioactive isotopes that can be used to label agents of the present invention include, but are not limited to Technetium^{99m}, Carbon¹¹, Oxygen¹⁵, Nitrogen¹³, Rubidium⁸², Gallium⁶⁷, Gallium⁶⁸, Yttrium⁹⁰, Molybdenum⁹⁹, Iodine^{123,124,131} Fluorine¹⁸, Phosphorus³², Copper⁶², Thallium²⁰¹, Copper⁶⁴, Copper⁶², Indium¹¹¹ and Xenon¹³³.

Radioisotopes are typically synthesized by a cyclotron, which accelerates subatomic particles from an ion source along a circular orbit. Particle acceleration inside a chamber is controlled by two alternating electromagnetic fields. These accelerated particles can gain energy and collide with a target at close to the speed of light. Bombardment of particles against the target results in unstable, radioactive isotopes, which are then attached to the agents of the present invention. Alternatively, commercially available radioisotopes are widely used (e.g., Amersham Int. Buckinghamshire, UK) and may be conjugated onto the agents of the present invention by chemical synthesis techniques well known in the art. Typically, the half-lives of radioactive agents used in imaging are relatively short, and thus many cyclotrons are key features of radiotracer detectors.

Another example of a detectable moiety that can be used for the present invention for use in both in-vivo and ex-vivo analysis of mitochondria is a fluorescent probe such as a fluorescent chemical or fluorescent polypeptide. Examples of fluorescent probes that can be used to label the agents of the present invention include but are not limited to the fluorescent chemicals, fluorescin (FITC), Cascade blue, Lucifer yellow, Fluor X, Red 613, X-Rhodamine and tetramethylrhodamine isothiocyanate (TRITC). Examples of fluorescent polypeptides include those belonging to the green fluorescent protein family, including the green fluorescent protein, the yellow fluorescent protein, the cyan fluorescent protein and the red fluorescent protein as well as their enhanced derivatives. Many fluorescent probes and polypeptides are commercially available from such Companies as Amersham Int. Buckinghamshire, UK, Molecular Probes - Invitrogen, Oregon, U.S. and BD Biosciences Clontech). Alexa Fluor 488 and Alexa Fluor 594 conjugates of anti-COX subunit I are also available for direct staining of mitochondria (catalogue numbers A21296, A21297)

Fluorescent activity includes emission of radiation, generally light, from a material during illumination by radiation of usually higher frequency or from the impact of electrons. For example, the fluorescent polypeptides and fluorescent probes can emit light of a characteristic wavelength when excited by light, which is generally of a characteristic and different wavelength than that used to generate the emission. Fluorescent polypeptides and probes also include those in which the chromophore is formed autocatalytically and does not require addition of a substrate to induce fluorescence. The fluorescent polypeptide or fluorescent probe can be, for example, one that fluoresces in the near-infrared (NIR) region (in the range of 600-1100 nm), after excitation in the far-red range of visible light wavelengths. The fluorescent polypeptide may also fluoresce in the far red region such as the fluorescent polypeptide Cy 5.5 and the Alexa Fluor® 700 fluorescent dye. The fluorescent polypeptide or fluorescent probe can also fluoresce in the blue region such as FITC, TRITC and Cy2. Since antibodies and other proteins absorb light having wavelengths up to about 310 nm, the fluorescent moieties should be selected to have substantial absorption at wavelengths above 310 nm and preferably above 400 nm.

The detectable moiety can also be an enzyme (e.g., cromogenic enzymes). Chromogenic enzymes, in the presence of a suitable substrate, may generate chromogenic products which may be used as a detectable moiety. Such enzymes include but are not limited to alkaline phosphatase, β-galactosidase, β-D-glucoronidase (GUS) and the like. Enzyme linked antibodies are commercially available (e.g., Jackson ImmunoResearch Laboratories and Sigma Aldrich).

Another detectable moiety for the detection of mitochondria is an epitope tag. This is a unique polypeptide sequence to which a specific antibody can bind without cross reacting with other cellular antigens. Epitope tags include a Myc tag, a Flag tag, a His tag, a Leucine tag, an IgG tag and the like. Epitope tagged antibodies are commercially available from such Companies as BioVision Inc. Exton, England and Sigma Aldrich, St. Louis, MO.

When both the mitochondrial agent and detectable moiety are polypeptides, the chimeric peptides thereof may be produced by recombinant means or may be chemically synthesized by, for example, the stepwise addition of one or more amino acid residues in defined order using solid phase peptide synthetic techniques. Examples of polypeptide moieties that can be linked to peptidic mitochondrial agents (e.g., antibodies) using recombinant DNA technology include fluorescent polypeptides, phosphorescent polypeptides, enzymes and epitope tags. Expression vectors can be designed to fuse proteins encoded by the heterologous nucleic acid insert to fluorescent polypeptides. For example, antibodies can be expressed from an expression vector fused with a green fluorescent protein (GFP)-like polypeptide. A wide variety of vectors are commercially available that fuse proteins encoded by heterologous nucleic acids to the green fluorescent protein from Aequorea victoria ("GFP"), the yellow fluorescent protein and the red fluorescent protein and their variants (e.g., Evrogen). In these systems, the fluorescent polypeptide is entirely encoded by its amino acid sequence and can fluoresce without requirement for cofactor or substrate. Expression vectors that can be employed to fuse proteins encoded by the heterologous nucleic acid insert to epitope tags are commercially available (e.g., BD Biosciences, Clontech).

Alternatively, chemical attachment of a detectable moiety to polypeptide can use any suitable chemical linkage, direct or indirect, as via a peptide bond (when the detectable moiety is a polypeptide), or via covalent bonding to an intervening linker element, such as a linker peptide or other chemical moiety, such as an organic polymer. Such chimeric peptides may be linked via bonding at the carboxy (C) or amino (N) termini of the peptides, or via bonding to internal chemical groups such as straight, branched or cyclic side chains, internal carbon or nitrogen atoms, and the like. Such modified peptides can be easily identified and prepared by one of ordinary skill in the art, using well known methods of peptide synthesis and/or covalent linkage of peptides. Description of fluorescent labeling of antibodies is provided in details in U.S. Pat. Nos. 3,940,475, 4,289,747, and 4,376,110.

As mentioned, measuring a quantitative difference in the number of mitochondria or a mitochondrial component as well as measuring an alteration in a mitochondrial characteristic between the prostate cell of the test subject and a control prostate cell can be effected ex-vivo (e.g. in vitro) or in vivo according to this aspect of the present disclosure. It will be appreciated that in-vivo detection of the cancer cells may be advantageous since it negates the need of performing an invasive procedure. It also allows for the direct localization of the cancerous cells. Another advantage of in vivo detection is that generally, an in vivo procedure can ascertain whether the cells are cancerous or not, much quicker than an ex vivo procedure.

Examples of agents which can be administered in vivo and which are selective for the mitochondria and thus can be used in the present invention are chemicals such as lipophilic cations, including, but not limited to, tetrofosmin and [MIBI]6, commonly known as sestamibi, where MIBI is 2-methoxy isobutyl isonitrile. These agents typically enter the cell via passive diffusion across plasma and mitochondrial membranes. Without being bound by theory, it is suggested that these lipophilic cations accumulate within the mitochondria and cytoplasm of cells on the basis of electrical potentials generated across the membrane bilayers. At equilibrium they are sequestered largely within mitochondria by a large negative transmembrane potential. Generally, these agents are fixed intracellularly as long as cell membrane integrity is intact and nutrient blood flow persists. These agents are typically radiolabeled so that they can be imaged in vivo. Thus, typical radioactive lipophilic cations that are selective for the mitochondria include Tc⁹⁹Sestamibi and ^{99m}Tc-tetrofosmin.

The short half-life of Tc⁹⁹ permits administration of high doses of Tc⁹⁹sestamibi yielding high imaging quality in a short acquisition time. It has been shown that more than 90 % of Tc⁹⁹sestamibi is taken up by mitochondria in an energy dependent manner. This uptake generally increases with the number of mitochondria present. In addition to mitochondrial activity, the presence of Tc⁹⁹sestamibi is dependent upon its delivery through the bloodstream to the region of the body being imaged. Tc⁹⁹Sestamibi is commercially available as Cardiolite®, or Miraluma® (Bristol Myers Squib - Medical Imaging) and Tc⁹⁹-tetrofosmin is commercially available as Myoview, (Amersham Int.). As illustrated in Figures 4A-T and 5A-E, ex-vivo SPECT scanning using Tc⁹⁹Sestamibi of an intact prostate identified tumor areas as corroborated by whole mount histopathology.

Other chemicals that selectively accumulate in the mitochondria, can be administered in vivo and are suitable for use in the present invention, include but are not limited to 2-fluoro-2-deoxyglucose, diacetyl-bis(N4-methylthiosemicarbazone) and 1,4,8,11-tetraazacyclotetradecane-N,N',N",N"'-tetraacetic acid - octreotide. The radiolabeled derivatives of these agents that can be used in the present invention are ¹⁸fluorine-labeled-2-fluoro-2-deoxyglucose (commercially available from such Companies as Health Imaging Isotopes), ⁶²Cu-diacetyl-bis(N4-methylthiosemicarbazone) - (ATSM), Tc99-triphenylalkylphosphonium and ⁶⁴Cu-1,4,8,11-tetraazacyclotetradecane-N,N',N",N"'-tetraacetic acid - octreotide.

Other agents that may be administered in vivo are polypeptide agents, such as for example, mitochondria specific antibodies or fragments thereof.

Antibody agents that recognize human mitochondrial specific proteins are commercially available, e.g., from Molecular Probes - Invitrogen (California), including monoclonal antibodies raised against the Oxidative phosphorylation (OxPhos) complex (monoclonal Antibodies Specific for OxPhos Complex IV - COX and monoclonal Antibodies Specific for Complexes I, II, III and V) as detailed herein below in Table I:

**Table 1- Invitrogen-Molecular probes:**

| ***Catalogue number*** | ***Oxidative complex*** | ***Subunit specificity*** |
|---|---|---|
| A21344 | I | 39,000-dalton subunit |
| A21343 | I | 30,000-dalton subunit |
| A31857 | I | 20,000-dalton subunit |
| A21359 | I | 17,000-dalton subunit |
| A31856 | I | 15,000-dalton subunit |
| A11142 | II | 70,000-dalton subunit |
| A21345 | II | 30,000-dalton subunit |
| A21362 | III | Core 1 subunit |
| A11143 | III | Core 2 subunit |
| A21346 | III | FeS subunit |
| A6403 | IV (COX) | Subunit I |
| A6404 | IV (COX) | Subunit II |
| A21347 | IV (COX) | Subunit IV |
| A21348 | IV (COX) | Subunit IV |
| A21363 | IV (COX) | Subunit Va |
| A21349 | IV (COX) | Subunit Vb |
| A21365 | IV (COX) | Subunit VIa-L |
| A21366 | IV (COX) | Subunit VIb |
| A6401 | IV (COX) | Subunit VIc |
| A21367 | IV (COX) | Subunit VIIa-H/L |
| A21368 | IV (COX) | Subunit VIIb |
| A21350 | V (ATPase) | α-subunit |
| A21351 | V (ATPase) | β-subunit |
| A21353 | V (ATPase) | d subunit |
| A21354 | V (ATPase) | OSCP subunit |
| A21355 | V (ATPase) | Inhibitor protein |

Other commercially available (e.g., Molecular Probes - Invitrogen, California) mitochondrial specific monoclonal antibodies include those specific for Mitochondrial porin (Catalogue numbers A21317 and A31855) and those specific for pyruvate dehydrogenase (Catalogue numbers A21323, A31853, A21324, A21325, A21326).

The term "antibody" as used in this invention includes intact molecules as well as functional fragments thereof, such as Fab, F(ab')2, and Fv that are capable of binding to the specific mitochondrial proteins. Smaller antibody fragments may be advantageous over whole antibodies since they are able to penetrate tissue more readily and are more rapidly cleared from the body. This is especially relevant for the in-vivo use of mitochondrial specific antibodies. This was recently shown for the V_{HH}s fragment [Cortez-Retamozo V. et al., Int. J. Cancer 2002, 98:456-462]. Also, an additional advantage of antibody fragments is that they may be produced in bacteria or yeasts.

Suitable Antibody fragments for practicing the present invention include a complementarity-determining region (CDR) of an immunoglobulin light chain (referred to herein as "light chain"), a complementarity-determining region of an immunoglobulin heavy chain (referred to herein as "heavy chain"), a variable region of a light chain, a variable region of a heavy chain, a light chain, a heavy chain, an Fd fragment, and antibody fragments comprising essentially whole variable regions of both light and heavy chains such as an Fv, a single chain Fv, an Fab, an Fab', and an F(ab')2.

Functional antibody fragments comprising whole or essentially whole variable regions of both light and heavy chains are defined as follows:
(i) Fv, defined as a genetically engineered fragment consisting of the variable region of the light chain and the variable region of the heavy chain expressed as two chains;
(ii) single chain Fv ("scFv"), a genetically engineered single chain molecule including the variable region of the light chain and the variable region of the heavy chain, linked by a suitable polypeptide linker.
(iii) Fab, a fragment of an antibody molecule containing a monovalent antigen-binding portion of an antibody molecule which can be obtained by treating whole antibody with the enzyme papain to yield the intact light chain and the Fd fragment of the heavy chain which consists of the variable and CH1 domains thereof;
(iv) Fab', a fragment of an antibody molecule containing a monovalent antigen-binding portion of an antibody molecule which can be obtained by treating whole antibody with the enzyme pepsin, followed by reduction (two Fab' fragments are obtained per antibody molecule); and
(v) F(ab')2, a fragment of an antibody molecule containing a monovalent antigen-binding portion of an antibody molecule which can be obtained by treating whole antibody with the enzyme pepsin (i.e., a dimer of Fab' fragments held together by two disulfide bonds).

Methods of generating antibodies (i.e., monoclonal and polyclonal) are well known in the art. Antibodies may be generated via any one of several methods known in the art, which methods can employ induction of in-vivo production of antibody molecules, screening of immunoglobulin libraries [Orlandi D.R. et al., 1989. Proc. Natl. Acad. Sci. U. S. A. 86:3833-3837; Winter G. et al., 1991. Nature 349:293-299] or generation of monoclonal antibody molecules by continuous cell lines in culture. These include, but are not limited to, the hybridoma technique, the human B-cell hybridoma technique, and the Epstein-Barr virus (EBV)-hybridoma technique [Kohler G. et al., 1975. Nature 256:495-497; Kozbor D. et al., 1985. J. Immunol. Methods 81:31-42; Cote RJ. et al., 1983. Proc. Natl. Acad. Sci. U. S. A. 80:2026-2030; Cole SP. et al., 1984. Mol. Cell. Biol. 62:109-120].

In cases where target antigens are too small to elicit an adequate immunogenic response when generating antibodies in-vivo, such antigens (haptens) can be coupled to antigenically neutral carriers such as keyhole limpet hemocyanin (KLH) or serum albumin (e.g., bovine serum albumin (BSA)) carriers [see, for example, US. Pat. Nos. 5,189,178 and 5,239,078]. Coupling a hapten to a carrier can be effected using methods well known in the art. For example, direct coupling to amino groups can be effected and optionally followed by reduction of the imino linkage formed. Alternatively, the carrier can be coupled using condensing agents such as dicyclohexyl carbodiimide or other carbodiimide dehydrating agents. Linker compounds can also be used to effect the coupling; both homobifunctional and heterobifunctional linkers are available from Pierce Chemical Company, Rockford, Ill**.** The resulting immunogenic complex can then be injected into suitable mammalian subjects such as mice, rabbits, and the like. Suitable protocols involve repeated injection of the immunogen in the presence of adjuvants according to a schedule which boosts production of antibodies in the serum. The titers of the immune serum can readily be measured using immunoassay procedures which are well known in the art.

The antisera obtained can be used directly or monoclonal antibodies may be obtained as described hereinabove.

Antibody fragments can be obtained using methods well known in the art. [see, for example, Harlow and Lane, "Antibodies: A Laboratory Manual", Cold Spring Harbor Laboratory, New York, (1988)]. For example, antibody fragments according to the present invention can be prepared by proteolytic hydrolysis of the antibody or by expression in E. coli or mammalian cells (e.g., Chinese hamster ovary cell culture or other protein expression systems) of DNA encoding the fragment.

Alternatively, antibody fragments can be obtained by pepsin or papain digestion of whole antibodies by conventional methods. As described hereinabove, an (Fab')2 antibody fragments can be produced by enzymatic cleavage of antibodies with pepsin to provide a 5S fragment. This fragment can be further cleaved using a thiol reducing agent, and optionally a blocking group for the sulfhydryl groups resulting from cleavage of disulfide linkages to produce 3.5S Fab' monovalent fragments. Alternatively, enzymatic cleavage using pepsin produces two monovalent Fab' fragments and an Fc fragment directly. Ample guidance for practicing such methods is provided in the literature of the art [for example, refer to: Goldenberg, U.S. Pat. Nos. 4,036,945 and 4,331,647; Porter, RR., 1959. Biochem. J. 73:119-126]. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

As described hereinabove, an Fv is composed of paired heavy chain variable and light chain variable domains. This association may be noncovalent [see, for example, Inbar et al., 1972. Proc. Natl. Acad. Sci. USA. 69:2659-62]. Alternatively, as described hereinabove the variable domains can be linked to generate a single chain Fv by an intermolecular disulfide bond, or alternately, such chains may be cross-linked by chemicals such as glutaraldehyde.

Preferably, the Fv is a single chain Fv. Single chain Fv's are prepared by constructing a structural gene comprising DNA sequences encoding the heavy chain variable and light chain variable domains connected by an oligonucleotide encoding a peptide linker. The structural gene is inserted into an expression vector, which is subsequently introduced into a host cell such as E. coli. The recombinant host cells synthesize a single polypeptide chain with a linker peptide bridging the two variable domains. Ample guidance for producing single chain Fv's is provided in the literature of the art [for example, refer to: Whitlow and Filpula, 1991. Methods 2:97-105; Bird et al., 1988. Science 242:423-426; Pack et al., 1993. Bio/Technology 11:1271-77; and Ladner et al., U.S. Pat. No. 4,946,778].

Isolated complementarity determining region peptides can be obtained by constructing genes encoding the complementarity determining region of an antibody of interest. Such genes may be prepared, for example, by RT-PCR of mRNA of an antibody-producing cell. Ample guidance for practicing such methods is provided in the literature of the art [for example, refer to Larrick and Fry, 1991. Methods 2:106-10].

It will be appreciated that for human therapy, humanized antibodies are preferably used. Humanized forms of non human (e.g., murine) antibodies are genetically engineered chimeric antibodies or antibody fragments having-preferably minimal-portions derived from non human antibodies. Humanized antibodies include antibodies in which complementary determining regions of a human antibody (recipient antibody) are replaced by residues from a complementarity determining region of a non human species (donor antibody) such as mouse, rat or rabbit having the desired functionality. In some instances, Fv framework residues of the human antibody are replaced by corresponding non human residues. Humanized antibodies may also comprise residues which are found neither in the recipient antibody nor in the imported complementarity determining region or framework sequences. In general, the humanized antibody will comprise substantially all of at least one, and typically two, variable domains, in which all or substantially all of the complementarity determining regions correspond to those of a non human antibody and all, or substantially all, of the framework regions correspond to those of a relevant human consensus sequence. Humanized antibodies optimally also include at least a portion of an antibody constant region, such as an Fc region, typically derived from a human antibody [see, for example, Jones et al., 1986. Nature 321:522-525; Riechmann et al., 1988. Nature 332:323-329; and Presta, 1992. Curr. Op. Struct. Biol. 2:593-596].

Methods for humanizing non human antibodies are well known in the art. Generally, a humanized antibody has one or more amino acid residues introduced into it from a source which is non human. These non human amino acid residues are often referred to as imported residues which are typically taken from an imported variable domain. Humanization can be essentially performed as described [see, for example: Jones et al., 1986. Nature 321:522-525; Riechmann et al., 1988. Nature 332:323-327; Verhoeyen et al., 1988. Science 239:1534-1536; U.S. Pat. No. 4,816,567] by substituting human complementarity determining regions with corresponding rodent complementarity determining regions. Accordingly, such humanized antibodies are chimeric antibodies, wherein substantially less than an intact human variable domain has been substituted by the corresponding sequence from a non human species. In practice, humanized antibodies may be typically human antibodies in which some complementarity determining region residues and possibly some framework residues are substituted by residues from analogous sites in rodent antibodies.

Human antibodies can also be produced using various techniques known in the art, including phage display libraries [see, for example, Hoogenboom and Winter, 1991. J. Mol. Biol. 227:381; Marks et al., 1991. J. Mol. Biol. 222:581; Cole et al., "Monoclonal Antibodies and Cancer Therapy", Alan R. Liss, pp. 77 (1985); Boerner et al., 1991. J. Immunol. 147:86-95]. Humanized antibodies can also be made by introducing sequences encoding human immunoglobulin loci into transgenic animals, e.g., into mice in which the endogenous immunoglobulin genes have been partially or completely inactivated. Upon antigenic challenge, human antibody production is observed in such animals which closely resembles that seen in humans in all respects, including gene rearrangement, chain assembly, and antibody repertoire. Ample guidance for practicing such an approach is provided in the literature of the art [for example, refer to: U.S. Pat. Nos. 5,545,807, 5,545,806, 5,569,825, 5,625,126, 5,633,425, and 5,661,016; Marks et al., 1992. Bio/Technology 10:779-783; Lonberg et al., 1994. Nature 368:856-859; Morrison, 1994. Nature 368:812-13; Fishwild et al., 1996. Nature Biotechnology 14:845-51; Neuberger, 1996. Nature Biotechnology 14:826; Lonberg and Huszar, 1995. Intern. Rev. Immunol. 13:65-93].

Once antibodies are obtained, they may be tested for activity, for example via ELISA.

For in vivo use, antibodies are generally either radiolabeled or fluorescent labeled. A variety of methods are known in the art for radiolabeling antibodies [e.g., as described in U.S. Pat. No. 5,985,240; U.S. Pat. No. 4,361,544 and U.S. Pat. No. 4,427,646]. Particular methods of radiolabeling will depend on the radioisotope used.

Radiolabeled antibodies may be commercially produced by Companies such as Metabion, Planegg-Martinsried.

Fluorescent labeled mitochondrial antibodies are also commercially available from Companies such as Molecular Probes, Invitrogen. For example, Molecular Probes offer a red-fluorescent Alexa Fluor 594 conjugate of anti-PDH E1α subunit antibody, as well as the green-fluorescent Alexa Fluor 488 conjugate of anti-PDH E2 subunit antibody. However, for in vivo use, it is preferable to use an antibody conjugated to Alexa Fluor 750 because biological tissues are relatively transparent to excitation light in the 700-800 nm spectral range.

Other examples of polypeptide agents that can be used in vivo include the proteins avidin, strepavidin and nutravidin. Avidin is a highly cationic 66,000-dalton glycoprotein with an isoelectric point of about 10.5. Streptavidin is a nonglycosylated 52,800-dalton protein with a near-neutral isoelectric point. Nutravidin is a deglycosylated form of avidin. All of these proteins have a very high affinity and selectivity for biotin, each capable of binding four biotins per molecule. Since endogenously biotinylated proteins in mammalian cells are present almost exclusively in mitochondria, where biotin synthesis occurs, labeled avidin, nutravidin and streptavidin can be used for the selective staining of mitochondria. For in vivo use it is preferable to use radiolabeled or fluorescent labeled avidin derivatives. A wide range of fluorescent labeled avidin derivatives for in vivo use in the present invention are available from Molecular Probes, Invitrogen. Fluorescin labeled avidin has successfully been administered in vivo in the detection of a biotinylated kidney [Hoya K. et al., Drug Delivery, 1 October 2001, vol. 8, no. 4, pp. 215-222(8)]

Another example of a mitochondria-specific agent that can be used in vivo in the present invention is a polynucleotide which is able to bind by base pairing to a nucleic acid sequence (i.e., DNA or RNA) specifically found in the mitochondria.

There are three types of oligonucleotide probes that may be used for the detection of mitochondrial RNA molecules - single stranded DNA probes, double stranded DNA probes and RNA probes.

Examples of mitochondrial oligonucleotides are discussed in U.S. Pat. Appl. No. 20050026167 to Birch-Machin.

The term "oligonucleotide" refers to a single stranded or double stranded oligomer or polymer of ribonucleic acid (RNA) or deoxyribonucleic acid (DNA) or mimetics thereof. This term includes oligonucleotides composed of naturally-occurring bases, sugars and covalent internucleoside linkages (e.g., backbone) as well as oligonucleotides having non-naturally-occurring portions which function similarly to respective naturally-occurring portions.

Oligonucleotides designed according to the teachings of the present invention can be generated according to any oligonucleotide synthesis method known in the art such as enzymatic synthesis or solid phase synthesis. Equipment and reagents for executing solid-phase synthesis are commercially available from, for example, Applied Biosystems. Any other means for such synthesis may also be employed; the actual synthesis of the oligonucleotides is well within the capabilities of one skilled in the art and can be accomplished via established methodologies as detailed in, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988) and "Oligonucleotide Synthesis" Gait, M. J., ed. (1984) utilizing solid phase chemistry, e.g. cyanoethyl phosphoramidite followed by deprotection, desalting and purification by for example, an automated trityl-on method or HPLC.

The oligonucleotide of the present disclosureare typically at least 17, at least 18, at least 19, at least 20, at least 22, at least 25, at least 30 or at least 40, bases specifically hybridizable with mitochondrial sequences described hereinabove.

It will be appreciated that therapeutic oligonucleotides may further include base and/or backbone modifications as described herein below, which may increase bioavailability, therapeutic efficacy and reduce cytotoxicity.

For example, the oligonucleotides of the present disclosuremay comprise heterocylic nucleosides consisting of purines and the pyrimidines bases, bonded in a 3' to 5' phosphodiester linkage.

Preferably used oligonucleotides are those modified in either backbone, internucleoside linkages or bases, as is broadly described herein under.

Specific examples of preferred oligonucleotides useful according to this aspect of the present disclosureinclude oligonucleotides containing modified backbones or non-natural internucleoside linkages. Oligonucleotides having modified backbones include those that retain a phosphorus atom in the backbone, as disclosed in U.S. Pat. NOs: 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466, 677; 5,476,925; 5,519,126; 5,536,821; 5,541,306; 5,550,111; 5,563,253; 5,571,799; 5,587,361; and 5,625,050.

Preferred modified oligonucleotide backbones include, for example, phosphorothioates, chiral phosphorothioates, phosphorodithioates, phosphotriesters, aminoalkyl phosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms can also be used.

Alternatively, modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts, as disclosed in U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623, 070; 5,663,312; 5,633,360; 5,677,437; and 5,677,439.

Other oligonucleotides which can be used according to the present disclosure, are those modified in both sugar and the internucleoside linkage, i.e., the backbone, of the nucleotide units are replaced with novel groups. The base units are maintained for complementation with the appropriate polynucleotide target. An example for such an oligonucleotide mimetic, includes peptide nucleic acid (PNA). A PNA oligonucleotide refers to an oligonucleotide where the sugar-backbone is replaced with an amide containing backbone, in particular an aminoethylglycine backbone. The bases are retained and are bound directly or indirectly to aza nitrogen atoms of the amide portion of the backbone. United States patents that teach the preparation of PNA compounds include, but are not limited to, U.S. Pat. Nos. 5,539,082; 5,714,331; and 5,719,262. Other backbone modifications, which can be used in the present disclosure, are disclosed in U.S. Pat. No: 6,303,374.

Oligonucleotides of the present disclosuremay also include base modifications or substitutions. As used herein, "unmodified" or "natural" bases include the purine bases adenine (A) and guanine (G), and the pyrimidine bases thymine (T), cytosine (C) and uracil (U). Modified bases include but are not limited to other synthetic and natural bases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Further bases include those disclosed in U.S. Pat. No: 3,687,808, those disclosed in The Concise Encyclopedia Of Polymer Science And Engineering, pages 858-859, Kroschwitz, J. I., ed. John Wiley & Sons, 1990, those disclosed by Englisch et al., Angewandte Chemie, International Edition, 1991, 30, 613, and those disclosed by Sanghvi, Y. S., Chapter 15, Antisense Research and Applications, pages 289-302, Crooke, S. T. and Lebleu, B., ed., CRC Press, 1993. Such bases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2°C. [Sanghvi YS et al. (1993) Antisense Research and Applications, CRC Press, Boca Raton 276-278] and are presently preferred base substitutions, even more particularly when combined with 2'-O-methoxyethyl sugar modifications.

It is possible to radiolabel all types of oligonucleotides with ¹⁸Fluorine, ³⁵S, ³H, ¹⁴C and ⁹⁹Tc. Isotopic phosphorus is unfavorable because its very short half life makes analysis difficult. Another disadvantage with oligonucleotides labeled on the 3' or 5' terminus with ³²P is that the label will be rapidly hydrolyzed by exonucleases that are abundant in the blood. ³⁵S has a higher energy, and therefore is easier to detect at low levels. Tritium is a relatively weak isotope, limiting levels of detection and analytical options. Radiolabeled sulfur obviously lends itself perfectly to use with phosphorothioate (PS) oligonucleotides. Although a single radioactive phosphorothioate link can be added to any non-PS oligonucleotide, an overt change to the molecular composition is made which defeats one advantage of using isotopes. Tritium, on the other hand, can be placed in any oligonucleotide, including phosphorothioates, with no change to molecular structure. Other radioisotopes that can be used to label oligonucleotides include, but are not limited to ¹⁸Fluorine, ¹⁴C and ⁹⁹Tc.

Oligonucleotides can also be fluorescence labeled. Fluorescent labeled dUTPS are commercially available (e.g., fluorescein 12-dideoxyuridine-5'-triphosphate oligodeoxyribonucleotides - Boehringer Mannheim (Germany) and may be incorporated in the oligonucleotide using the enzyme terminal deoxynucleotidyl transferase (Life Technologies, Inc.) Fluorescent labeled oligonucleotides such as Rhodamine X labeled oligonucleotides may be ordered commercially from Companies such as Takara Shuzo (Kyoto, Japan) and Metabion, Planegg-Martinsried. Phosphorescent dUTPs may also be prepared [De Haas, R., 1999, Journal of Histochemistry and Cytochemistry, Vol. 47, 183-196] and used to produce phosphorescent labeled oligonucleotides.

The prior art teaches of a number of delivery strategies which can be used to efficiently deliver oligonucleotides into a wide variety of cell types [see, for example, Luft J Mol Med 76: 75-6 (1998); Kronenwett et al., Blood 91: 852-62 (1998); Rajur et al., Bioconjug Chem 8: 935-40 (1997); Lavigne et al., Biochem Biophys Res Commun 237: 566-71 (1997) and Aoki et al., (1997) Biochem Biophys Res Commun 231: 540-5 (1997)].

Agents of the present invention may be administered to the subject per se or in a pharmaceutical composition which includes carriers or vehicles. Useful carriers and vehicles include, but are not limited to, ion exchangers, alumina, aluminum stearate, lecithin, serum proteins such as albumin, buffer substances such as phosphate, glycine, sorbic acid, potassium sorbate, tris(hydroxymethyl)amino methane ("TRIS"), partial glyceride mixtures of fatty acids, water, salts or electrolytes, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polypropylene block co-polymers, sugars such as glucose, and suitable cryoprotectants. Techniques for formulation and administration of drugs may be found in "Remington's Pharmaceutical Sciences," Mack Publishing Co., Easton, PA, latest edition.

The pharmaceutical compositions of the mitochondrial agents described herein above can be in the form of a sterile injectable preparation. The possible vehicles or solvents that can be used to make injectable preparations include water, Ringer's solution, and isotonic sodium chloride solution, and 5% D-glucose solution (D5W). In addition, oils such as mono- or di-glycerides and fatty acids such as oleic acid and its derivatives can be used.

Agents with their detectable moieties for use in the in vivo detection of mitochondria can be administered, for example, parenterally, by inhalation, topically, rectally, nasally, buccally, or via an implanted reservoir. For example, an antibody can also be administered via catheters or through a needle directly to the prostate. The term parenteral as used herein includes percutaneous, subcutaneous, intravascular (e.g., intravenous), intramuscular, or intrathecal injection or infusion techniques. Preferably the mitochondrial agent with its detectable moiety is administered intravenously.

The amount of an agent used for in in-vivo mitochondrial analysis and the duration of the imaging study will depend upon the label attached to the agent, the body mass of the patient, the nature and severity of the condition being treated, the nature of therapeutic treatments which the patient has undergone, and on the idiosyncratic responses of the patient. For example, one of ordinary skill in the art will recognize that the amount of a radioactive agent used in vivo mitochondrial analysis will depend on the radioisotope used for the labeling of the mitochondrial agent. Different radioisotopes display different pharmacokinetic properties, such as elimination, clearance from and/or accumulation in biological tissues, and half-life.

Preferably, a detectably effective amount of the agent of the invention is administered to a subject. This is defined as an amount sufficient to yield an acceptable image using equipment, which is available for clinical use. A detectably effective amount of the agent of the invention may be administered in more than one injection. Ultimately, the attending physician will decide the amount of agent to administer to each individual patient and the duration of the imaging study. For example if the radioactive agent is sestamibi, a detectable effective amount will typically be between about 10 and about 30 mCi. Optimization of such factors is well within the level of skill in the art.

Analyzing mitochondria according to the method of this aspect of the present invention is effected by imaging the agents. In vivo imaging techniques include, but are not limited to radioimaging, fluoresence imaging, biophotonic imaging and magnetic resonance imaging.

Radioimaging is defined as the imaging of the spatial distribution of a radioactive agent which accumulates in a particular cell or sub-cellular component or cellular fluid. Radioimaging can be performed both in vivo and ex-vivo. Preferably, when the imaging is performed in vivo the radioimaging technique is nuclear imaging. Generally nuclear imaging is aimed at visualizing molecular and cellular processes occurring in living tissues. As such, nuclear imaging typically serves as a medical tool for measuring signals of molecules within the tissue and thus for generating quantitative images of physiological, biochemical and pharmacological function.

Single Photon Emission Computed Tomography (SPECT) and Positron Emission Tomography (PET) are representative examples of nuclear imaging techniques, which have been effectively used in a plurality of applications and can also be used for the in vivo imaging of mitochondria in the present invention. Both are techniques which image a radioactively labeled agent (e.g., SPECT can detect Tc⁹⁹Sestamibi and PET can detect ¹⁸fluorine-labeled-2-fluoro-2-deoxyglucose). The uptake of the radioactive agent is measured over time and used to obtain information about mitochondrial quantity. While PET and SPECT rely on similar principles to produce their images, important differences in instrumentation, radiochemistry, and experimental applications are dictated by inherent differences in their respective physics of photon emission.

Unstable nuclides that possess an excess number of protons generally take one of two approaches in an effort to reduce their net nuclear positivity. In one radioactive decay scheme, a proton is converted to a neutron and a particle called a positron is emitted [Hoffman, E. J., and Phelps, M. E. New York: Raven Press; 1986: 237-286; Sorenson, J. A., and Phelps, M. E. Philadelphia: W. B. Saunders; 1987]. Of identical mass but opposite charge, positrons are the antimatter equivalent of electrons. When ejected from the nucleus, a positron collides with an electron, resulting in the annihilation of both particles and the release of energy. Two gamma photons are produced, each of equivalent energy and opposite trajectory (generally 180 degrees apart).

The unique spatial signature of back-to-back photon paths is exploited by PET scanners in locating the source of an annihilation event, a method known as coincidence detection [Hoffman, E. J., and Phelps, M. E. New York: Raven Press; 1986: 237-286; Links, J. M. New York: Raven Press; 1990: 37-50]. PET (and SPECT) scanners employ scintillation detectors made of dense crystalline materials (e.g., bismuth germanium oxide, sodium iodide, or cesium fluoride), that capture the high-energy photons and convert them to visible light. This brief flash of light is converted into an electrical pulse by an adjacent photomultiplier tube (PMT). The crystal and PMT together typically make up a radiation detector. A PET camera is constructed such that opposing detectors are electronically connected. Thus, when separate scintillation events in paired detectors coincide, an annihilation event is presumed to have occurred at some point along an imaginary line between the two. This information is used to reconstruct images using the principles of computed tomography. Conversely, single events are ignored. Although it is conceivable that two unrelated photons from spatially separate annihilation events might reach opposing detectors in unison, these accidental coincidences are much less frequent than true ones. Nevertheless, random coincidences constitute a source of background noise in PET images [Hoffman E J et al. J Comput Assist Tomogr (1981); 5:391-400; Hoffman, E. J., and Phelps, M. E. New York: Raven Press; (1986): 237-286; Links, J. M. New York: Raven Press; (1990): 37-50].

SPECT is another method of the present invention for radioimaging mitochondria. It can directly detect isotopes that decay by electron capture and/or gamma emissions. Certain proton-rich radioactive isotopes, such as ¹²³I and Tc⁹⁹ are capable of capturing an orbiting electron, transforming a proton to a neutron [Sorenson J A, and Phelps M E. Philadelphia: W. B. Saunders; 1987]. The resulting daughter nucleus often remains residually excited. This meta-stable arrangement subsequently dissipates, thereby achieving a ground state and producing a single gamma photon in the process. Because gamma photons are emitted directly from the site of decay, no comparable theoretical limit on spatial resolution exists for SPECT. However, instead of coincidence detection, SPECT utilizes a technique known as collimation [Jaszczak R J. Boca Raton: CRC Press; (1991): 93-118]. A collimator may be thought of as a lead block containing many tiny holes that is interposed between the subject and the radiation detector. Given knowledge of the orientation of a collimator's holes, the original path of a detected photon is linearly extrapolated and the image is reconstructed by computer-assisted tomography.

To gain the closest access to the prostate, a rectally inserted radioactive-emission-measuring probe is used. The present invention preferably may be employed together with the Radioactive-Emission Measurement Probe for the Prostate, described in WO 2005/118659 and WO 2005/119025. In this invention, the solid state detectors move with respect to the housing unit as opposed to the movement of the whole camera. This allows for enhanced prostate cancer imaging.

A gamma camera may also be used to detect the location and distribution of gamma-emitting radioisotopes.

In vivo fluorescence imaging, which can be used in the present invention to analyze mitochondria, may be carried out using a suitable fluorescence imaging camera or device. For the present invention, in vivo fluorescence imaging may be used to detect a fluoresecent labeled polypeptide (including antibodies and avidin derivatives) and fluorescent labeled polynucleotides.

A fluorescent imaging system useful in the practice of this invention typically includes three basic components: (1) a source of light of a wavelength suitable to cause the fluorescent polypeptide to fluoresce, (2) an apparatus for separating or distinguishing emissions from light used for fluorescent excitation, and (3) a detection system. [Weissleder et al., Nat. Biotechnol., 17:375-8, 1999].

The source of excitation light is generally a filtered light source or a laser with a defined bandwidth. The excitation light travels through body tissues. When it encounters a fluorescent molecule (i.e., a "contrast agent"), the excitation light is absorbed. The fluorescent molecule then emits light that has, for example, detectably different spectral properties (e.g., a slightly longer wavelength) from the excitation light.

The fluorescent technology utilized in the present invention may be near infa red (NIR) technology. This offers unique advantages for imaging pathology, because tissues and blood have a high transmittance in the near-infrared range (700-850 nm) as opposed to visible light, and neither water nor many naturally occurring fluorochromes absorbs significantly in this region. Thus, NIR light penetrates tissues more efficiently than visible light or photons in the infrared region. In addition, there is lower interference of scattered excitation with far-red light. As a result, the fluorescence signal excited in the deeper layers of tissue can be acquired (reviewed in Hawrysz and Sevick-Muraca, Neoplasia, 2:388-417, 2000). In general, images of tissues can be obtained at a depth of up to tens of centimeters, e.g., at least 1 cm, 2 cm, 3 cm, 4 cm, 5 cm, 6 cm, 7 cm, 8 cm, 9 cm, 10 cm, 12 cm, 15 cm, 18 cm, or 20 cm.

The light source provides monochromatic (or substantially monochromatic) light. When using NIR fluorescent polypeptides, the light source typically provides near infa red light. When using far-red fluorescent polypeptides, the light source typically provides far red light. The light source can equally provide violet light or blue light depending on the fluorescent polypeptide. The light source can be a suitably filtered white light, e.g., bandpass-filtered light from a broadband source. For example, light from a 150-watt halogen lamp can be passed through a suitable bandpass filter commercially available from Omega Optical (Brattleboro, Vt). The light source could also be a laser. See, e.g., Boas et al., Proc. Natl. Acad. Sci. USA, 91:4887-4891, 1994; Ntziachristos et al., Proc. Natl. Acad. Sci. USA, 97:2767-2772, 2000; Alexander, J. Clin. Laser Med. Surg., 9:416-418, 1991. Information on lasers for imaging can also be found on the Internet (e.g., at imds.com) and various other known sources. A high pass or bandpass filter (700 nm) can be used to separate optical emissions from excitation light. A suitable high pass or bandpass filter is commercially available from Omega Optical.

In general, the light detection system can include light-gathering/image-forming and light-detection/image-recording components. Although the light-detection system can be a single integrated device that incorporates both components, the light-gathering/image-forming and light-detection/image-recording components will be discussed separately. However, a recording device may simply record a single (time varying) scalar intensity instead of an image. For example, a catheter-based recording device can record information from multiple sites simultaneously (i.e., an image), or can report a scalar signal intensity that is correlated with location by other means (such as a radio-opaque marker at the catheter tip, viewed by fluoroscopy). A particularly useful light-gathering/image-forming component is an endoscope. Endoscopic devices and techniques that have been used for in vivo optical imaging of numerous tissues and organs, including peritoneum [Gahlen et al., J. Photochem. Photobiol., B 52:131-135, 1999], ovarian cancer [Major et al., Gynecol. Oncol., 66:122-132, 1997], colon [Mycek et al., Gastrointest. Endosc., 48:390-394, 1998; Stepp et al., Endoscopy, 30:379-386, 1998], bile ducts [Izuishi et al., Hepatogastroenterology, 46:804-807, 1999], stomach [Abe et al., Endoscopy 32:281-286, 2000], bladder [Kriegmair et al., Urol. Int., 63:27-31, 1999; Riedl et al., J. Endourol., 13:755-759, 1999], and brain [Ward, J. Laser Appl., 10:224-228, 1998] can be employed in the practice of the present invention. Fluorescence endoscopes are also known in the art [Bhunchet et al., Gastrointest. Endosc., 55, 562-571, 2002; Kobayashi et al., Cancer Lett., 165, 155-159, 2001]. One of skill in the art would be able to recognize and make any modifications that may be required, e.g., to optimize the emission and detection spectra of the device for use in imaging a particular organ or tissue region.

Any suitable light-detection/image-recording component, e.g., charge-coupled device (CCD) systems or photographic film, can be used in the invention. The choice of light-detection/image-recording component will depend on factors including type of light gathering/image forming component being used. Selecting suitable components, assembling them into an imaging system, and operating the system is within the ability of a person of ordinary skill in the art.

Additional information on oligonucleotide for in vivo imaging may be found on the Official web site of the European funded research program "OLIM" (oligoimaging.free.fr/olim/).

Magnetic Resonance (MR) imaging is another example of an imaging technique that may be used in vivo for this invention. It is based on the principle that hydrogen nuclei in a strong magnetic field absorb pulses of radiofrequency energy and emit them as radio waves which can be reconstructed into computerized images. Magnetic Resonance imaging typically offers both near-cellular (i.e. 50 micron) resolution and whole-body imaging capability.

In order to be visualized, cells are typically labeled with an intracellular marker that can be detected by MR imaging. Superparamagnetic iron oxide nanoparticles provide the highest sensitivity when used as MR contrast agent and are the most preferred contrast agent for use in the present invention. Gadolinum is another widely contrast agent that can be used for mitochondrial MR imaging. It is possible to attach these metal complexes to mitochondrial agent and thereby achieve enhanced mitochondrial imaging. Other examples of elements that may be used for mitochondrial magnetic resonance imaging include, but are not limited to terbium, tin, iron, or isotopes thereof [Schaefer et al., (1989) JACC 14, 472-480; Shreve et al., (1986) Magn. Reson. Med. 3, 336-340; Wolf, G L., (1984) Physiol. Chem. Phys. Med. NMR 16, 93-95; Wesbey et al., (1984) Physiol. Chem. Phys. Med. NMR 16, 145-155; Runge et al., (1984) Invest. Radiol. 19, 408-415]. The covalent attachment of gadolinium and gadolinium complexes to proteins is well known [Niemi et al., 1991 Invest Radiol. 1991 (7):674-80]. The preparation and guidelines for administration of such compounds is best determined by a skilled practitioner such as a physician or radiologist [Magnetic Resonance Imaging, American College of Radiology, standards. 1996].

Suitable mitochondrial agents for covalent attachment of an MRI contrast agent include, but are not limited to, peptides, such as antibodies, and nucleic acids such as oligonucleotides as discussed herein.

As will be appreciated by those in the art, the mitochondrial agent may be attached to the MR contrast agent in a number of different ways, and in a variety of configurations.

Alternatively, in vivo biophotonic imaging (Xenogen, Almeda, Calif.) may be utilized for in vivo imaging. This real-time in vivo imaging utilizes luciferase. The luciferase gene is incorporated into cells, microorganisms, and animals (e.g., as a fusion protein with a mitochondrial peptide). When active, it leads to a reaction that emits light. A CCD camera and software is used to capture the image and analyze it.

As mentioned, the present disclosurealso envisages detection of prostate cancer cells ex-vivo. Cells for ex-vivo analysis may be retrieved by a surgical biopsy. The surgical biopsy procedure may include any biopsy procedure presently known in the art. The sextant prostate biopsy is an exemplary method. It is preferable to use an imaging technique in conjunction with the surgical biopsy so as to improve its accuracy. An example of an imaging technique which can be used in the present disclosureis a transrectal ultrasound.

Once a portion of the prostate tissue has been isolated, prostate cells may be isolated using standard procedures. Alternatively, prostate tissue may be frozen and analyzed. Methods of freezing tissues are well known in the art. Subsequently, the prostate tissue or the isolated cells may then be processed according to the particular ex vivo method of detection of mitochondria. Prostate cells may be processed so as to remain intact or its membrane disintegrated (such as following lysis or sonication) so as to release the inner cell components as described herein below.

As used herein, "an intact prostate cell" refers to a prostate cell in which the cell membrane is intact and all the intracellular components are present. A "disintegrated prostate cell" is a cell in which the cell membrane is broken and the intracellular components are released.

One method of processing prostate cells so that they remain intact is by tryspsinization of either cultured cells or of cells from a prostate biopsy. Standard culturing techniques are also well known in the art. Cells may be fixed on slides using standard procedures for ex vivo analysis. Fixing acts to preserve cells in a reproducible and life-like manner, following their removal in a surgical biopsy procedure. Additionally, many intracellular mitochondrial components are soluble molecules that can be washed out and depleted by staining methods if fixing techniques are not applied. Fixation methods are well known in the art. These methods typically rely on either crosslinking agents, such as paraformaldehyde, or rapidly dehydrating agents, such as methanol.

Agents and methods which may be used specifically for ex-vivo analysis on prostate cells are described hereinbelow.

Mitochondrial dyes are an example of agents that may be used ex vivo for the present disclosure. A diverse array of cell permeant fluorescent dyes as well as perfused cell dyes, are capable of selectively associating with mitochondria in living cells such as prostate cells. Generally, mitochondrial specific dyes are attracted to the inner mitochondrial membrane potential that exists in the mitochondria as a result of the active extrusion of protons from the mitochondrial matrix to the intermembrane space via complexes of the electron transport chain.

Using mitochondrial specific dyes, either a change in mitochondrial quantity or a change in mitochondrial potential may be accurately measured. For measuring mitochondrial quantity, the mitochondrial dye typically accumulates in the mitochondria regardless of the mitochondrial membrane potential. Examples of membrane independent mitochondrial specific dyes known in the art include, but are not limited to MitoFluor Green, MitoFluor Red 5809, MitoTracker Red 580, MitoTracker Deep Red 633, nonyl acridine orange and MitoTracker Green FM, all of which are commercially available from Molecular probes. [For example see "Handbook of Fluorescent Probes and Research Chemicals" www.probes.com/handbook/sections/1202.html), Chapter 12 - Probes for Organelles]. Mitochondrial staining by nonyl acridine orange is attributed to binding to the lipid mitochondrial component, cardiolipin, (as described herein above) in the inner mitochondrial membrane.

As mentioned, mitochondrial dyes may also be used to measure a change in a mitochondrial characteristic, i.e. mitochondrial potential. Examples of mitochondrial dyes which may be used in this aspect of the present invention include, but are not limited to MitoTracker Orange CMTMRos, MitoTracker Orange CM-H₂TMRos, MitoTracker Red CMXRos, MitoTracker Red CM-H₂XRos, MitoTracker Red 580, MitoTracker Deep Red 633, MitoFluor Red 594, RedoxSensor Red CC-1 (2,3,4,5,6-pentafluorotetramethyldihydrorosamine, JC-1 probe (5,5',6,6'-tetrachloro-1,1',3,3'-tetraethylbenzimidazolylcarbocyanine iodide, Rhodamine 123, tetramethylrosamine, rhodamine 6G, tetramethylrhodamine methyl ester, tetramethylrhodamine ethyl ester, dihydrorhodamine, dihydrotetramethylrosamine, DiOC₂(3), DiOC₅(3), DiOC₆(3), DiSC₃(5), DiIC₁(5), DASPMI (4-Di-1-ASP), DASPEI and CoroNa Red Na⁺.

It will be appreciated that use of dyes (e.g. mitotracker dyes - Molecular Probes) that are retained in the mitochondria following fixation and permeabilization may be advantageous since the fluorescent staining pattern characteristic of live cells is retained during subsequent processing steps for immunocytochemistry, *in situ* hybridization or electron microscopy.

Mitochondrial dyes are typically added directly to live cultured prostate cells.

Both antibodies and avidin derivatives may be used in vivo and ex vivo although both are preferred for ex vivo examination.

Immunohistochemistry can be used for ex-vivo imaging antibody agents. According to this aspect of the present invention, a mitochondrial specific antibody is used to link a mitochondrial protein specifically to a detectable moiety so that it can be more readily seen with a microscope. Antibodies may be detected directly, or alternatively a secondary antibody may be included. The mitochondrial protein-specific antibodies may be enzyme linked or linked to fluorophores. Detection is by microscopy and subjective or automatic evaluation. If enzyme linked antibodies are employed, a colorimetric reaction may be required as further described herein below. It will be appreciated that immunohistochemistry is often followed by counterstaining of the cell nuclei using for example Hematoxyline or Giemsa stain.

In order to detect mitochondrial components of prostate cells, the ex vivo mitochondrial agent must have access to the intracellular compartment of the cell. Thus when needed, the cell should be permeabilized to allow entry of the mitochondrial agents. Permeabilization of prostate cells can typically be performed simultaneously with fixation or post-fixation. Numerous permeabilizing agents, such as detergents, organic solvents, etc. are well known to the skilled artisan, including saponin and methanol. Additionally, many peptides and toxins that render membranes permeable are known to the skilled artisan [e.g., Bussing et al., Cytometry, 1999, 37:133-9]. Additionally, physical methods may also be used to render cells permeable [e.g., Dent et al., Methods in Enzymology, 1995, 255:265-73; Pind et al., Methods in Enzymology, 1993, 221:222-34].

The time period that cells are permeabilized can be critical for staining of mitochondrial antigens. Cells may be permeabilized in 2% saponin or 80% methanol from 10 minutes to 12 hours, to several days, depending on the analyte of interest. Selecting an appropriate permeabilizing agent and optimizing the time period is well within the skill of the artisan, and should be performed empirically.

Fluorescence activated cell sorting (FACS) is a method for ex vivo quantitation of mitochondria or a mitochondrial component. It involves detection of a mitochondrial protein *in situ* in cells by mitochondria specific antibodies. The mitochondria specific antibodies are linked to fluorophores. Detection is by means of a cell sorting machine which reads the wavelength of light emitted from each cell as it passes through a light beam. This method may employ two or more antibodies simultaneously.

Enzyme linked immunosorbent assay (ELISA) may be used for the ex-vivo determination of the quantity of a particular mitochondrial protein in prostate cells. This method involves fixation of cells to a surface such as a well of a microtiter plate. Alternatively an extract is prepared from the cells, as described herein below which is used as as source of protein. A mitochondrial specific antibody coupled to an enzyme is applied and allowed to bind to the mitochondrial protein inside the cell. Presence of the antibody is then detected and quantitated by a colorimetric reaction employing the enzyme coupled to the antibody. Enzymes commonly employed in this method include horseradish peroxidase and alkaline phosphatase. If well calibrated and within the linear range of response, the amount of substrate present in the sample is proportional to the amount of color produced. A substrate standard is generally employed to improve quantitative accuracy.

The *in situ* activity assay may be employed for the ex-vivo determination of the activity of a particular mitochondrial enzyme in prostate cells. According to this method, a chromogenic substrate is applied on the cells containing an active mitochondrial enzyme and the enzyme catalyzes a reaction in which the substrate is decomposed to produce a chromogenic product visible by a light or a fluorescent microscope.

Mitochondrial-specific labeled polynucleotides may be detected ex vivo by, for example, in-situ hybridization.

Typically, polynucleotide probes require a hybridization buffer in order to base pair with their target. The hybridization buffer usually includes reagents such as formamide and salts (e.g., sodium chloride and sodium citrate) which enable specific hybridization of the DNA or RNA probes with their target RNA molecules *in situ* while avoiding non-specific binding of probe. Those skilled in the art are capable of adjusting the hybridization conditions (i.e., temperature, concentration of salts and formamide and the like) to specific probes and types of cells.

In situ RT-PCR staining is another method that may be used ex vivo to detect the presence of mitochondrial RNA. This method is described in Nuovo GJ, et al. [Intracellular localization of polymerase chain reaction (PCR)-amplified hepatitis C cDNA. Am J Surg Pathol. 1993, 17: 683-90] and Komminoth P, et al. [Evaluation of methods for hepatitis C virus detection in archival liver biopsies. Comparison of histology, immunohistochemistry, in situ hybridization, reverse transcriptase polymerase chain reaction (RT-PCR) and in situ RT-PCR. Pathol Res Pract. 1994, 190: 1017-25]. Briefly, the RT-PCR reaction is performed on fixed cells by incorporating detectable moieties, in this case labeled nucleotides, such as P³² labeled nucleotides to the PCR reaction. The reaction is carried out using specific *in situ* RT-PCR apparatus such as the laser-capture microdissection PixCell I LCM system available from Arcturus Engineering (Mountainview, CA).

Signal amplification methods may be used in conjugation with the above techniques for the ex-vivo analysis of the mitochondria of prostate cells. Signals may be amplified by using commercially-available amplifiers known in the art, which increase the signal to mitochondrial agent ratio.

As mentioned above, prostate cells may also be processed by removing the cell membrane so as to release the inner prostate cell components. Various prostate cell protein extracts may be prepared (e.g. whole cell protein extracts or mitochondrial protein extracts) and analyzed for an alteration in quantity and/or activity according to this aspect of the present invention. Mitochondrial protein extracts may be prepared using a mitochondrial extraction kit (e.g. from Imgenex, California), catalogue number 10082K.

Expression (i.e. quantity) and/or activity level of proteins expressed in the disintegrated prostate cells of the present invention can be determined using methods known in the arts as detailed herein below.

The in vitro activity assay is a method for the ex-vivo determination of the activity of a particular enzyme in cell extracts. In this method the activity of a particular mitochondrial enzyme is measured in a protein mixture extracted from the cells. The activity can be measured in a spectrophotometer well using colorimetric methods or can be measured in a non-denaturing acrylamide gel (i.e., activity gel). Following electrophoresis the gel is soaked in a solution containing a substrate and colorimetric reagents. The resulting stained band corresponds to the enzymatic activity of the protein of interest. If well calibrated and within the linear range of response, the amount of enzyme present in the sample is proportional to the amount of color produced. An enzyme standard is generally employed to improve quantitative accuracy.

As mentioned above, prostate cell protein extracts may also be used for the determination of the quantity of a particular prostate protein. For this purpose, a western blot may be performed. This method involves separation of a particular mitochondrial protein from other proteins by means of an acrylamide gel followed by transfer of the mitochondrial protein to a membrane (e.g., nylon or PVDF). Presence of the mitochondrial protein is then detected by specific antibodies, which are in turn detected by antibody binding reagents. Antibody binding reagents may be, for example, protein A, or other antibodies. Antibody binding reagents may be radiolabeled or enzyme linked as described hereinabove and hereinbelow.

Radio-immunoassay (RIA) may also be used for the ex-vivo determination of the quantity of a particular mitochondrial protein in cell extracts. In one version, this method involves precipitation of the desired mitochondrial protein (*i.e.,* the substrate) with a specific antibody and radiolabeled antibody binding protein (e.g., protein A labeled with I¹²⁵) immobilized on a precipitable carrier such as agarose beads. The number of counts in the precipitated pellet is proportional to the amount of substrate.

In an alternate version of the RIA, the protein extract is labeled (e.g. radiolabeled) and an unlabelled antibody binding protein is employed. Methods of labeling proteins are well known in the art. A protein extract containing an unknown amount of a particular mitochondrial protein is added in varying amounts. The decrease in precipitated counts from the labeled protein extract is proportional to the amount of mitochondrial protein in the added sample.

Additionally or alternatively, RNA may be extracted from the cells and analyzed for an alteration in quantity according to this aspect of the present invention.

Numerous methods of total RNA extraction are known in the art and include the use of guanidinium hydrochloride and cesium chloride [Glisin et al. (1974) Biochemistry 13: 2633; Ullrich et al. (1977) Science 196: 1313; Chomczynski et al. (1987) Anal. Biochem. 162: 156] or the use of guanidinium hydrochloride and organic solvents [Strohman et al. (1977) Cell 10: 265; McDonald et al. (1987) Meth. Enzymol. 152: 219]. Alternatively, RNA extraction kits are commercially available. For example, RNA STAT 60.RTM. (Tel-Test, Inc., Friendswood, Tex.), RNeasy.RTM. (QIAGEN), Tripure.RTM. (Boehringer Mannheim Biochemicals, Indianapolis, Ind.), Trizol (GIBCO Laboratories, Gaithersburg, Md.), and Tri Reagent.RTM. (Molecular Research Center, Inc., Cincinnati, Ohio).

Methods of isolating mitochondrial RNA have previously been described [Attardi et al., 1983, Methods Enzymol. 1983; 97:435-69; Stern and Newton, 1986, Methods Enzymol. 118: 488-496]. The quantity of mitochondrial RNA in the cells of the present invention can be determined using methods known in the arts.

For example, Northern Blot analysis may be performed for the quantitation of a particular mitochondrial RNA in a mixture of RNAs. An RNA sample is denatured by treatment with an agent (e.g., formaldehyde) that prevents hydrogen bonding between base pairs, ensuring that all the RNA molecules have an unfolded, linear conformation. The individual RNA molecules are then separated according to size by gel electrophoresis and transferred to a nitrocellulose or a nylon-based membrane to which the denatured RNAs adhere. The membrane is then exposed to labeled DNA probes. Probes may be labeled using radio-isotopes or enzyme linked nucleotides. Detection may be using autoradiography, colorimetric reaction or chemiluminescence.

Alternatively, RT-PCR analysis may be performed for the quantitation of a particular mitochondrial RNA in a mixture of RNAs This method is especially useful in quantitating relatively rare mitochondrial RNA molecules. First, RNA molecules are purified from the cells and converted into complementary DNA (cDNA) using a reverse transcriptase enzyme (such as an MMLV-RT) and primers such as, oligo dT, random hexamers or gene specific primers. Then by applying gene specific primers and Taq DNA polymerase, a PCR amplification reaction is carried out in a PCR machine. Those of skills in the art are capable of selecting the length and sequence of the gene specific primers and the PCR conditions (*i.e.,* annealing temperatures, number of cycles and the like) which are suitable for detecting specific RNA molecules. It will be appreciated that a semi-quantitative RT-PCR reaction can be employed by adjusting the number of PCR cycles and comparing the amplification product to known controls.

Both Northern blot and RT-PCR have successfully been used for analyzing human mitochondrial RNA [e.g. Pich et al., FEBS Lett. 2004 Jan 30;558(1-3):19-22].

Detectable moieties for the ex vivo detection of mitochondria that may be used in the present invention include radioisotopes, fluorescent polypeptides, phosphorescent molecules, chemiluminescent molecules, luminescent molecules, and fluorescent probes as described herein above for in vivo detectable moieties. Enzymes and epitope tags may also be used, but unlike radioisotopes and fluorescent probes they are preferred for ex vivo detection only.

Color imaging can detect changes in color of ex vivo systems. It is typically used for immunohistochemistry and in situ hybridization and generally employs an enzyme reaction. For immunohistochemistry, the antibody is enzyme linked. For in-situ hybridization, the oligonucleotide is enzyme linked. Color changes may be detected under a regular microscope. Table 1 below shows the colors that can be produced using particular enzymes and substrates.

**Table 2**

| ***enzyme,*** | ***substrate*** | ***abbreviation*** | ***Final color*** |
|---|---|---|---|
| Horseradish peroxidase | Diaminobenzidine | DAB | brown |
| | Diaminobenzidine with nickel enhancement. | DAB/Nickel | Gray/ Black |
| | 3-Amino 9-ethylcarbazole. | AEC | Red |
| | 4-Chloro-1-naphthol. | N/A | Blue |
| Alkaline phosphatase | Naphthol-AS-B1-phosphate/ fast red TR. | NABP/FR | Red |
| | Naphthol-AS-MX-phosphate/ fast red TR. | NAMP/FR | Red |
| | Naphthol-AS-B1-phosphate/ new fuschin. | NABP/NF | Red |
| | bromochloroindolyl phosphate/nitroblue tetrazolium. | BCIP/NBT | Purple |
| | 5-Bromo-4-chloro-3-indolyl-b-d-galactopyranoside. | BCIG | Blue |

Ex-vivo detection of radioactive agents generally includes the use of film and/or phoSphoimager screens (e.g. molecular dynamics or Fujifilm BAS 1500). The image of the exposed phosphoimager screen is analyzed using a phosphoimager.

Fluorescent microscopes may be used to image cells labeled with dyes or fluorescent labeled polypeptides (e.g. antibodies) and polynucleotides ex vivo. A wide range of fluorescent microscopes exist which are commercially available.

An ex-vivo technique for analyzing mitochondria which can also be included in the present disclosureis the direct use of microscopy to image mitochondria without the use of a detectable agent. Typically, the microscopy is electron microscopy. This technique has successfully been used for the analysis of mitochondria [e.g. Gunter F. et al., The Journal of Cell Biology, Vol 15, 489-501].

Detection of prostate cancer according to the present disclosureis preferably combined with other diagnosis procedures which are well known in the art and described in the Background section. This may provide a more accurate diagnosis of the disease and its stage.

It will be appreciated that the above described teachings of the present invention can also be used to stage prostate cancer. Typically, one or more cancer cells from a variety of patients are staged according to procedures well-known in the art, and the quantity of mitochondria or mitochondrial component or the mitochondrial characteristic therein is determined for each stage to obtain standards according to the methods described herein above. The mitochondrial data from the subject of interest are then compared to the standard mitochondrial data. By comparing the mitochondrial data per cell from the subject to the standard expression levels, it is possible to determine the stage of the tumor.

The method of the present disclosurecan also include a method of monitoring prostate cancer in a subject. One may monitor a subject to determine whether a preneoplastic lesion has become cancerous. One may also monitor a subject to determine whether a therapy, e.g., chemotherapy, radiotherapy or surgery, has decreased or eliminated the prostate cancer. The monitoring may determine if there has been a reoccurrence and, if so, determine its nature. Thus, a subject is monitored periodically for prostate cancer as described herein. If there is a change in mitochondrial or mitochondrial component quantity over time, or a change in mitochondrial characteristic in one direction, this can be associated with treatment failure, or conversion of a preneoplastic lesion to a cancerous lesion. One having ordinary skill in the art would recognize that a change in mitochondrial or mitochondrial component quantity over time or a change in a mitochondrial characteristic in the opposite direction, would be indicative of effective therapy or failure to progress to a neoplastic lesion.

Reagents for analyzing mitochondria or a mitochondrial component as described above can be included in a diagnostic kit for detecting prostate cancer. Such kit may include the imaging agent packed in a container along with buffers and stabilizers as well as instructions of using the kit and interpreting the results obtained. For large scale screening of a plurality of samples (ex-vivo), the diagnostic imaging reagent may be attached to a solid addressable support such as an array.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Current Protocols in Molecular Biology" Volumes I-III Ausubel, R. M., ed. (1994); Ausubel et al., "Current Protocols in Molecular Biology", John Wiley and Sons, Baltimore, Maryland (1989); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980). Other general references are provided throughout this document. The procedures therein are believed to be well known in the art and are provided for the convenience of the reader.

### EXAMPLE 1

### MATERIALS AND EXPERIMENTAL METHODS

Expression of a protein component of human mitochondria was analyzed immunohistochemically by the labelled streptavidin/ biotin method on formalin-fixed, paraffin embedded tissues. Sections of prostate tissue were dewaxed in xylene, and rehydrated through descending graded alcohols and rinsed in distilled water. Sections were then incubated with primary antibodies. Antigen retrieval was carried out using a pressure cooker and EDTA buffer (pH=8) 110 °C, for 6min. Following blocking of endogenous peroxidase activity with 1% H₂O₂ (30 min) and non-specific antibody binding with 1% bovine albumin in NaCl/Pi for 30 min, sections were incubated with primary antibodies.

Incubation was carried out with anti-mitochondria monoclonal antibody (BioGenex) in 1:80 dilutions for 1 h incubation at room temperature. Sections were subsequently incubated with prediluted biotinylated link antibody (DAKO LSAB 2 Kit, Dako Corp. CA, U.S.A.) for 30 min followed by streptavidin-horseradish peroxidase conjugate one (DAKO LSAB 2 Kit, Dako Corp. CA, U.S.A.) for 30 min. After washing, peroxidase activity was detected using 3,3'-diaminobenzidine as chromogen with H₂O₂ as substrate. The sections were counter stained with haematoxylin, dehydrated, cleared in xylene and mounted. For recording of the immunohistochemical staining a semi-quantitative and subjective grading, considering both the proportion of tumor cells showing positive reaction and intensity of staining was used. The proportion of stained cells (PP) was graded as the percent of tumor cells stained. Staining intensity (SI) was graded as: - -no staining, 1 - weak staining, 2 - moderate staining, 3 - strong staining.

### EXPERIMENTAL RESULTS

Sixteen samples of prostate cancer tissue samples from prostatectomy, TURP, or needle biopsy of various Gleason grades (5-10) were analyzed.

The samples were stained with anti-mitochondrial monoclonal antibodies. The staining pattern was granular as is characteristic for mitochondrial staining. In normal prostate tissue (BPH) staining was absent to minimal. An average of 5.6 % (5-20 %) of normal cells were stained (Figure 1a) with an intensity of weak (1) and occasionally moderate (2). An average of 71.2 % (50-90 %) of tumor cells were stained (Figure 1b) with an intensity of moderate (2) to strong (3) (Figure 1c.). Areas with absence of staining may be partially due to technical artifacts. Staining was completely absent (-) in stroma, blood vessels, inflammation and nerves (Figure 2).

### EXAMPLE 2

### MATERIALS AND EXPERIMENTAL METHODS

Twelve prostate cancer patients were intravenously administered with 25mCi Tc⁹⁹Sestamibi 1-2 hours prior to prostate excision. Intact prostates were excised using surgical procedures (Radical Retro pubic Prostatectomy, Radical Cystectomy or Open Prostatectomy) - illustrated in Figure 3. The intact prostate was imaged by SPECT using a standard dual head gamma camera (millennium, GE) 6° angular step and 40sec/step. Imaging results were reviewed using both transaxial (Figures 4I-N and Figures 5C-D) and coronal views (Figures 4O-T and Figure 5E). The nuclear images were processed automatically. The designated green lines seen in Figures 4I-M and 4O-R and 5C and 5E were produced following an automatic search for maximal intensity voxel. Contours were determined at 90 % maximal intensity. Following imaging, prostate tissue was sliced transversely from bladder neck (base) to apex. Whole mount histopathology was carried out as described in Example 1.

### EXPERIMENTAL RESULTS

As illustrated in Figures 4A-T and 5A-E, SPECT scanning using Tc⁹⁹Sestamibi of an intact prostate from two patients identified tumor areas as corroborated by histopathology. The tumor areas correlated precisely using the two techniques.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

## Claims

1. A method of obtaining information suitable for detecting presence or absence of early stage prostate cancer, being stage I or II prostate cancer, in the prostate of a subject after in vivo administration of a Tc^{99m}Sestamibi radioimaging agent to the subject, the method comprising reviewing and processing previously obtained results of radioimaging at least a portion of the prostate of the subject; ex vivo histochemically staining at least one prostate cell of the prostate with a reagent capable of binding to, or accumulating in, mitochondria; analyzing mitochondria or a mitochondrial component of said at least one prostate cell; corroborating said results of radioimaging with said ex vivo histochemical staining; and measuring an alteration in quantity and/or characteristic of said mitochondria or mitochondrial component with respect to a normal prostate cell of the prostate.

2. The method of claim 1, wherein said mitochondrial component is selected from the group consisting of mitochondrial protein, mitochondrial glycoprotein, mitochondrial lipid, mitochondrial peptide and mitochondrial nucleic acid.

3. The method of claim 2, wherein said mitochondrial protein is an enzyme or a structural protein.

4. The method of claim 1, wherein said characteristic of said mitochondria is selected from the group consisting of mitochondrial size, mitochondrial mass, mitochondrial potential and mitochondrial volume.

5. The method of claim 1, wherein said reagent is labeled with a detectable moiety.

6. The method of claim 5, wherein said detectable moiety is selected from the group consisting of a polypeptide and a chemical.

7. The method of claim 6, wherein said polypeptide is selected from the group consisting of an enzyme, a fluorescent polypeptide and an epitope.

8. The method of claim 6, wherein said chemical is a radioactive isotope, a phosphorescent chemical, a chemiluminescent chemical and a fluorescent chemical.

9. The method of claim 8, wherein said radioactive isotope can be detected by radioimaging.

10. The method of claim 8, wherein said radioactive isotope is selected from the group consisting of Technetium^{99m}, Carbon¹¹ Oxygen¹⁵, Nitrogen¹³, Rubidium⁸², Gallium⁶⁷, Gallium⁶⁸, Yttrium⁹⁰, Molybdenum⁹⁹, Iodine^{123,124,131}, Fluorine¹⁸, Phosphorus³², Copper⁶², Thallium²⁰¹, Copper⁶⁴, Copper⁶², Indium¹¹¹, Xenon¹³³.

11. The method of claim 1, wherein said reagent is selected from the group consisting of Tc⁹⁹Sestamibi, Tc⁹⁹-tetrofosmin, Tc⁹⁹-triphenylalkylphosphonium, ¹⁸fluorine-labeled-2-fluoro-2-deoxyglucose, ⁶²Cu-diacetyl-bis(N4-methylthiosemicarbazone) and ⁶⁴Cu-1,4,8,11-tetraazacyclotetradecane-N,N',N",N'''-tetraacetic acid - octreotide.

12. The method of claim 1, further comprising imaging said at least one ex vivo histochemically stained prostate cell.

13. The method of claim 12, wherein said imaging is selected from the group consisting of radioimaging, fluorescence imaging, color imaging, biophotonic imaging and magnetic resonance imaging.

14. The method of claim 1 or claim 13, wherein said radioimaging is selected from the group consisting of single photon emission computed tomography (SPECT), positron emission tomography (PET) and gamma cameras.

15. The method of claim 1 or claim 14, wherein said radioimaging provides one or more images of one or more sections or slices of the prostate.

16. The method of claim 1, wherein said at least one prostate cell is intact.

17. The method of claim 1, wherein said at least one prostate cell is disintegrated.

18. The method of claim 1, wherein said reagent is selected from the group consisting of a chemical, a dye, a peptide, a polypeptide and a polynucleotide.

19. The method of claim 18, wherein said dye is administered directly into said at least one prostate cell.

20. The method of claim 18, wherein said dye is membrane potential-independent.

21. The method of claim 20, wherein said membrane potential-independent dye is selected from the group consisting of nonyl acridine orange, MitoTracker Green FM, MitoFluor Green and MitoFluor Red 589.

22. The method of claim 18, wherein said dye is membrane potential-dependent.

23. The method of claim 22, wherein said membrane potential-dependent dye is selected from the group consisting of MitoTracker Orange CMTMRos, MitoTracker Orange CM-H₂TMRos, MitoTracker Red CMXRos, MitoTracker Red CM-H₂XRos, MitoTracker Red 580, MitoTracker Deep Red 633, MitoFluor Red 594, RedoxSensor Red CC-1 (2,3,4,5,6-pentafluorotetramethyldihydrorosamine, JC-1 probe (5,5',6,6'-tetrachloro-1,1',3,3'-tetraethylbenzimidazolylcarbocyanine iodide, Rhodamine 123, tetramethylrosamine, rhodamine 6G, tetramethylrhodamine methyl ester, tetramethylrhodamine ethyl ester, dihydrorhodamine, dihydrotetramethylrosamine, DiOC₂(3), DiOC₅(3), DiOC₆(3), DiSC₃(5), DiIC₁(5), DASPMI (4-Di-1-ASP), DASPEI and CoroNa Red Na⁺.

24. The method of claim 18, wherein said polypeptide is selected from the group consisting of an antibody, an avidin, and a derivative thereof.

25. The method of claim 24, wherein said avidin derivative is selected from the group consisting of avidin, strepavidin and nutravidin.

26. The method of claim 1, used for monitoring early stage prostate cancer in a subject.

27. The method of claim 1, wherein said corroborating comprises analyzing photographs of said radioimaging and said ex vivo histochemical staining.

28. The method of claim 1, wherein said corroborating comprises correlating areas of said radioimaged prostate portion and said at least one ex vivo histochemically stained prostate cell.

## Patentansprüche

1. Verfahren zum Erhalten von Informationen, die zum Nachweisen des Vorhandenseins oder Nichtvorhandenseins von Prostatakrebs im Frühstadium, wobei es sich um Prostatakrebs in Stadium I oder II handelt, in der Prostata eines Subjekts nach einer In-vivo-Verabreichung eines Tc^{99m}Sestamibi-Szintigrafiemittels an das Subjekt geeignet sind, wobei das Verfahren ein Überprüfen und Verarbeiten von früher erhaltenen Ergebnissen der Szintigrafie wenigstens eines Teils der Prostata des Subjekts; histochemisches Ex-vivo-Färben mindestens einer Prostatazelle der Prostata mit einem Reagens, das zum Binden an oder Akkumulieren in Mitochondrien imstande ist; Analysieren von Mitochondrien oder einer mitochondrialen Komponente der mindestens einen Prostatazelle; Bekräftigen der Szintigrafie-Ergebnisse mit der histochemischen Ex-vivo-Färbung; und Messen einer Änderung der Quantität und/oder Charakteristik der Mitochondrien oder mitochondrialen Komponente in Bezug auf eine normale Prostatazelle der Prostata umfasst.

2. Verfahren nach Anspruch 1, wobei die mitochondriale Komponente aus der Gruppe bestehend aus mitochondrialem Protein, mitochondrialem Glycoprotein, mitochondrialem Lipid, mitochondrialem Peptid und mitochondrialer Nucleinsäure ausgewählt wird.

3. Verfahren nach Anspruch 2, wobei das mitochondriale Protein ein Enzym oder ein Strukturprotein ist.

4. Verfahren nach Anspruch 1, wobei die Charakteristik der Mitochondrien aus der Gruppe bestehend aus Mitochondriengröße, Mitochondrienmasse, Mitochondrienpotenzial und Mitochondrienvolumen ausgewählt wird.

5. Verfahren nach Anspruch 1, wobei das Reagens mit einer nachweisbaren Komponente markiert wird.

6. Verfahren nach Anspruch 5, wobei die nachweisbare Komponente aus der Gruppe bestehend aus einem Polypeptid und einer Chemikalie ausgewählt wird.

7. Verfahren nach Anspruch 6, wobei das Polypeptid aus der Gruppe bestehend aus einem Enzym, einem fluoreszierenden Polypeptid und einem Epitop ausgewählt wird.

8. Verfahren nach Anspruch 6, wobei die Chemikalie ein radioaktives Isotop, eine phosphoreszierende Chemikalie, eine chemilumineszierende Chemikalie und eine fluoreszierende Chemikalie ist.

9. Verfahren nach Anspruch 8, wobei das radioaktive Isotop durch Szintigrafie nachgewiesen werden kann.

10. Verfahren nach Anspruch 8, wobei das radioaktive Isotop aus der Gruppe bestehend aus Technetium^{99m}, Kohlenstoff¹¹, Sauerstoff¹⁵, Stickstoff¹³, Rubidium⁸², Gallium67, Gallium⁶⁸, Yttrium⁹⁰, Molybdän⁹⁹, Iod^{123,124,131}, Fluor¹⁸, Phosphor³², Kupfer⁶², Thallium²⁰¹, Kupfer⁶⁴, Kupfer⁶², Indium¹¹¹, Xenon¹³³ ausgewählt wird.

11. Verfahren nach Anspruch 1, wobei das Reagens aus der Gruppe bestehend aus Tc⁹⁹Sestamibi, Tc⁹⁹-Tetrofosmin, Tc⁹⁹-Triphenylalkylphosphonium, ¹⁸Fluor-markierter 2-Fluor-2-deoxyglucose, ⁶²Cu-Diacetyl-bis(N4-methylthiosemicarbazon) und ⁶⁴Cu-1,4,8,11-Tetraazacyclotetradecan-N,N',N",N"'-tetraessigsäure - Octreotid - ausgewählt wird.

12. Verfahren nach Anspruch 1, ferner umfassend ein Abbilden der mindestens einen ex vivo histochemisch gefärbten Prostatazelle.

13. Verfahren nach Anspruch 12, wobei das Abbilden aus der Gruppe bestehend aus Szintigrafie, Fluoreszenzbildgebung, Farbbildgebung, biophotonischer Bildgebung und Magnetresonanzbildgebung ausgewählt wird.

14. Verfahren nach Anspruch 1 oder 13, wobei die Szintigrafie aus der Gruppe bestehend aus Einzelphotonen-Emissions-Computertomografie (SPECT), Positronen-Emissions-Tomografie (PET) und Gammakameras ausgewählt wird.

15. Verfahren nach Anspruch 1 oder 14, wobei die Szintigrafie ein oder mehrere Bilder eines oder mehrerer Schnitte oder einer oder mehrerer Scheiben der Prostata bereitstellt.

16. Verfahren nach Anspruch 1, wobei die mindestens eine Prostatazelle intakt ist.

17. Verfahren nach Anspruch 1, wobei die mindestens eine Prostatazelle zersetzt ist.

18. Verfahren nach Anspruch 1, wobei das Reagens aus der Gruppe bestehend aus einer Chemikalie, einem Farbstoff, einem Peptid, einem Polypeptid und einem Polynucleotid ausgewählt wird.

19. Verfahren nach Anspruch 18, wobei der Farbstoff direkt in die mindestens eine Prostatazelle verabreicht wird.

20. Verfahren nach Anspruch 18, wobei der Farbstoff vom Membranpotenzial unabhängig ist.

21. Verfahren nach Anspruch 20, wobei der vom Membranpotenzial unabhängige Farbstoff aus der Gruppe bestehend aus Nonylacridin-Orange, MitoTracker Green FM, MitoFluor Green und MitoFluor Red 589 ausgewählt wird.

22. Verfahren nach Anspruch 18, wobei der Farbstoff vom Membranpotenzial abhängig ist.

23. Verfahren nach Anspruch 2, wobei der vom Membranpotenzial abhängige Farbstoff aus der Gruppe bestehend aus MitoTracker Orange CMTMRos, MitoTracker Orange CM-H2TMRos, MitoTracker Red CMXRos, MitoTracker Red CM-H2XRos, MitoTracker Red 580, MitoTracker Deep Red 633, MitoFluor Red 594, RedoxSensor Red CC-1 (2,3,4,5,6-Pentafluortetramethyldihydrorosamin, JC-1 Sonde (5,5',6,6'-Tetrachlor-1,1',3,3'-tetraethylbenzimidazolylcarbocyaniniodid, Rhodamin 123, Tetramethylrosamin, Rhodamin 6G, Tetramethylrhodaminmethylester, Tetramethylrhodaminethylester, Dihydrorhodamin, Dihydrotetramethylrosamin, DiOC₂(3), DiOC₅(3), DiOC₆(3), DiSC₃(5), DiIC₁(5), DASPMI (4-Di-1-ASP), DASPEI und CoroNa Red Na⁺ ausgewählt wird.

24. Verfahren nach Anspruch 18, wobei das Polypeptid aus der Gruppe bestehend aus einem Antikörper, einem Avidin und einem Derivat davon ausgewählt wird.

25. Verfahren nach Anspruch 24, wobei das Avidin aus der Gruppe bestehend aus Avidin, Strepavidin und Nutravidin ausgewählt wird.

26. Verfahren nach Anspruch 1, das zum Überwachen von Prostatakrebs in einem Subjekt im Frühstadium verwendet wird.

27. Verfahren nach Anspruch 1, wobei das Bekräftigen ein Analysieren von Fotografien der Szintigrafie und der histochemischen Ex-vivo-Färbung umfasst.

28. Verfahren nach Anspruch 1, wobei das Bekräftigen ein Korrelieren des der Szintigrafie unterzogenen Prostatateils und der mindestens einen ex vivo histochemisch gefärbten Prostatazelle umfasst.

## Revendications

1. Procédé d'obtention d'informations appropriées pour détecter la présence ou l'absence du cancer de la prostate à un stade précoce, à savoir le cancer de la prostate au stade I ou II, dans la prostate d'un sujet après l'administration *in vivo* d'un agent de radio-imagerie Tc^{99m} Sestamibi au sujet, le procédé comprenant l'examen et le traitement des résultats précédemment obtenus de la radio-imagerie d'au moins une partie de la prostate du sujet ; la coloration histochimique *ex vivo* d'au moins une cellule prostatique de la prostate avec un réactif capable de se lier aux, ou de s'accumuler dans, les mitochondries ; l'analyse des mitochondries ou d'un composant mitochondrial de ladite au moins une cellule prostatique ; la corroboration desdits résultats de la radio-imagerie avec ladite coloration histochimique *ex vivo ;* et la mesure d'une altération de la quantité et/ou d'une caractéristique desdites mitochondries ou dudit composant mitochondrial par rapport à une cellule prostatique normale de la prostate.

2. Procédé selon la revendication 1, dans lequel ledit composant mitochondrial est sélectionné dans le groupe consistant en une protéine mitochondriale, une glycoprotéine mitochondriale, un lipide mitochondrial, un peptide mitochondrial et un acide nucléique mitochondrial.

3. Procédé selon la revendication 2, dans lequel ladite protéine mitochondriale est une enzyme ou une protéine structurale.

4. Procédé selon la revendication 1, dans lequel ladite caractéristique desdites mitochondries est sélectionnée dans le groupe consistant en la taille mitochondriale, la masse mitochondriale, le potentiel mitochondrial et le volume mitochondrial.

5. Procédé selon la revendication 1, dans lequel ledit réactif est marqué avec un fragment détectable.

6. Procédé selon la revendication 5, dans lequel ledit fragment détectable est sélectionné dans le groupe consistant en un polypeptide et un agent chimique.

7. Procédé selon la revendication 6, dans lequel ledit polypeptide est sélectionné dans le groupe consistant en une enzyme, un polypeptide fluorescent et un épitope.

8. Procédé selon la revendication 6, dans lequel ledit agent chimique est un isotope radioactif, un agent chimique phosphorescent, un agent chimique chimiluminescent et un agent chimique fluorescent.

9. Procédé selon la revendication 8, dans lequel ledit isotope radioactif peut être détecté par radio-imagerie.

10. Procédé selon la revendication 8, dans lequel ledit isotope radioactif est sélectionné dans le groupe consistant en le technétium^{99m}, le carbone¹¹, l'oxygène¹⁵, l'azote¹³, le rubidium⁸², le gallium⁶⁷, le gallium⁶⁸, l'yttrium⁹⁰, le molybdène⁹⁹, l'iode^{123,124,131}, le fluor¹⁸, le phosphore³², le cuivre⁶², le thallium²⁰¹, le cuivre⁶⁴, le cuivre⁶², l'indium¹¹¹, le xénon¹³³.

11. Procédé selon la revendication 1, dans lequel ledit réactif est sélectionné dans le groupe consistant en le Tc⁹⁹ Sestamibi, le Tc⁹⁹-tétrofosmine, le Tc⁹⁹-triphénylalkylphosphonium, le 2-fluoro-2-désoxyglucose marqué au fluor¹⁸, le Cu⁶²-diacétyl-bis-(N4-méthylthiosemicarbazone) et le Cu⁶⁴-acide 1,4,8,11-tétraazacyclotétradécane-N,N',N",N"'-tétraacétique-octréotide.

12. Procédé selon la revendication 1, comprenant en outre une imagerie de ladite au moins une cellule prostatique colorée par histochimie *ex vivo.*

13. Procédé selon la revendication 12, dans lequel ladite imagerie est sélectionnée dans le groupe consistant en la radio-imagerie, l'imagerie par fluorescence, l'imagerie couleur, l'imagerie biphotonique et l'imagerie par résonance magnétique.

14. Procédé selon la revendication 1 ou la revendication 13, dans lequel ladite radio-imagerie est sélectionnée dans le groupe consistant en la tomographie d'émission monophotonique (SPECT), la tomographie par émission de positons (TEP) et des gamma-caméras.

15. Procédé selon la revendication 1 ou la revendication 14, dans lequel ladite radio-imagerie permet d'obtenir une ou plusieurs images d'une ou de plusieurs sections ou coupes de la prostate.

16. Procédé selon la revendication 1, dans lequel ladite au moins une cellule prostatique est intacte.

17. Procédé selon la revendication 1, dans lequel ladite au moins une cellule prostatique est désintégrée.

18. Procédé selon la revendication 1, dans lequel ledit réactif est sélectionné dans le groupe consistant en un agent chimique, un colorant, un peptide, un polypeptide et un polynucléotide.

19. Procédé selon la revendication 18, dans lequel ledit colorant est administré directement dans ladite au moins une cellule prostatique.

20. Procédé selon la revendication 18, dans lequel ledit colorant est indépendant du potentiel de membrane.

21. Procédé selon la revendication 20, dans lequel ledit colorant indépendant du potentiel de membrane est sélectionné dans le groupe consistant en la nonyl acridine orange, le MitoTracker Green FM, le MitoFluor Green et le MitoFluor Red 589.

22. Procédé selon la revendication 18, dans lequel ledit colorant est dépendant du potentiel de membrane.

23. Procédé selon la revendication 22, dans lequel ledit colorant dépendant du potentiel de membrane est sélectionné dans le groupe consistant en le MitoTracker Orange CMTMRos, le MitoTracker Orange CM-H₂TMRos, le MitoTracker Red CMXRos, le MitoTracker Red CM-H₂XRos, le MitoTracker Red 580, le MitoTracker Deep Red 633, le MitoFluor Red 594, le RedoxSensor Red CC-1 (2,3,4,5,6-pentafluorotétraméthyldihydrorosamine, une sonde JC-1 (iodure de 5,5',6,6'-tétrachloro-1,1',3,3'-tétraéthylbenzimidazolylcarbocyanine), la Rhodamine 123, la tétraméthylrosamine, la rhodamine 6G, l'ester méthylique de tétraméthylrhodamine, l'ester éthylique de tétraméthylrhodamine, la dihydrorhodamine, la dihydrotétraméthylrosamine, le DiOC₂(3), le DiOC₅(3), le DiOC₆(3), le DiSC₃(5), le DiIC₁(5), le DASPMI (4-Di-1-ASP), le DASPEI et le CoroNa Red Na⁺.

24. Procédé selon la revendication 18, dans lequel ledit polypeptide est sélectionné dans le groupe consistant en un anticorps, une avidine, et un dérivé de celle-ci.

25. Procédé selon la revendication 24, dans lequel ledit dérivé d'avidine est sélectionné dans le groupe consistant en l'avidine, la streptavidine et la neutravidine.

26. Procédé selon la revendication 1, utilisé pour surveiller le cancer de la prostate à un stade précoce chez un sujet.

27. Procédé selon la revendication 1, dans lequel ladite corroboration comprend l'analyse de photographies de ladite radio-imagerie et de ladite coloration histochimique *ex vivo.*

28. Procédé selon la revendication 1, dans lequel ladite corroboration comprend la corrélation de régions de ladite partie de la prostate ayant été soumise à la radio-imagerie et de ladite au moins une cellule prostatique colorée par histochimie *ex vivo.*
